(19) Europäisches Patentamt
European Patent Office
Office européen des brevets

(11) Publication number: 0 322 616 A2

# (12) EUROPEAN PATENT APPLICATION

(21) Application number: 88120594.2

(22) Date of filing: 09.12.88

(51) Int. Cl.[4]: C07D 401/04 , C07D 209/48 , C07D 471/22 , C07D 213/81 , C07D 401/14 , A01N 43/40 , A01N 43/90 , A01N 43/38 , A01N 43/50 , A01N 53/00 , //(C07D471/22,235:00,221:00, 209:00)

(30) Priority: 31.12.87 US 139996

(43) Date of publication of application: 05.07.89 Bulletin 89/27

(84) Designated Contracting States: AT BE CH DE ES FR GB GR IT LI LU NL SE

(71) Applicant: AMERICAN CYANAMID COMPANY
1937 West Main Street P.O. Box 60
Stamford Connecticut 06904-0060(US)

(72) Inventor: Doehner, Robert Francis, Jr.
733 Windsor-Perrineville Road
East Windsor New Jersey 08520(US)
Inventor: Ladner, David William
99 Limewood Drive
Hamilton Square New Jersey 08690(US)
Inventor: Finn, John Michael
43 Montana Avenue
Trenton New Jersey 08619(US)

(74) Representative: Wächtershäuser, Günter, Dr.
Tal 29
D-8000 München 2(DE)

(54) **Novel 5(and/or 6)substituted 2-(2-imidazolin-2-yl)nicotinic acids, esters and salts, useful as herbicidal agents and nivel intermediates for the preparation of said nicotinic acids, esters and salts.**

(57) There are provided novel 5(and/or 6)substituted 2-(2-imidazolin-2-yl) and 2-(2-imidazolidinyl)nicotinic acids, esters and salts, which are useful as herbicidal agents. There are also provided novel substituted pyrrolopyridine acetonitriles, pyrrolopyridine acetamides, imidazopyrrolopyridinediones and carbamoylnicotinic acid esters, that are useful as intermediates for the preparation of the above said herbicidal agents.

EP 0 322 616 A2

# NOVEL 5(AND/OR 6) SUBSTITUTED 2-(2-IMIDAZOLIN-2-YL)NICOTINIC ACIDS, ESTERS AND SALTS, USEFUL AS HERBICIDAL AGENTS AND NOVEL INTERMEDIATES FOR THE PREPARATION OF SAID NICOTINIC ACIDS, ESTERS AND SALTS

## SUMMARY OF THE INVENTION

This invention relates to novel, herbicidal, 5(and/or 6)substituted 2-(2-imidazolin-2-yl)nicotinic acids, esters and salts depicted by formula I and 5(and/or 6)substituted 2-(2-imidazolidinyl)nicotinic acids, esters and salts depicted by formula II,

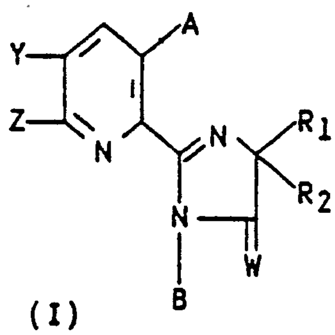

(I)

and

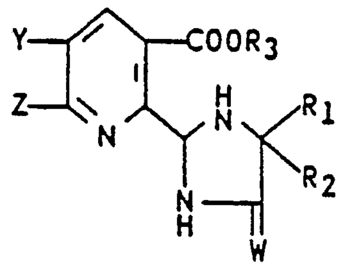

(II) ;

wherein

$R_1$ is $C_1$-$C_4$ alkyl;

$R_2$ is $C_1$-$C_4$ alkyl or $C_3$-$C_6$ cycloalkyl; and when $R_1$ and $R_2$ are taken together with the carbon to which they are attached they may represent $C_3$-$C_6$ cycloalkyl optionally substituted with methyl;

A is $COOR_3$, $CONHR_6$, CHO, $CH_2OH$, $COCH_3$, $COC_6H_5$, CN, $CH_3$, CH=NOH, $CH_2COOH$, CONHOH, $CH_2CH_2COOH$, $CHR_8OH$,

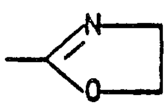

or

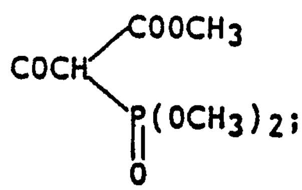

$R_3$ is hydrogen,

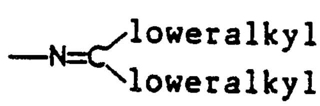

$C_1$-$C_{12}$ alkyl optionally substituted with one of the following groups: $C_1$-$C_3$ alkoxy, $C_1$-$C_3$ alkylthio, halogen, hydroxyl, $C_3$-$C_6$ cycloalkyl, benzyloxy, furyl, phenyl, halophenyl, loweralkylphenyl, loweralkoxycarbonyl, nitrophenyl, carboxyl, loweralkoxycarbonyl, cyano or triloweralkylammonium halide;

$C_3$-$C_{12}$ alkenyl optionally substituted with one of the following groups: $C_1$-$C_3$ alkoxy, phenyl, halogen or loweralkoxycarbonyl or with two $C_1$-$C_3$ alkoxy groups or two halogen groups;

$C_3$-$C_6$ cycloalkyl optionally substituted with one or two $C_1$-$C_3$ alkyl groups;

$C_3$-$C_{16}$ alkynyl optionally substituted with one or two $C_1$-$C_3$ alkyl groups; or

a cation;

$R_6$ is hydrogen, hydroxyl, $C_3$-alkenyl, $C_3$-alkynyl or $C_1$-$C_4$ alkyl optionally substituted with one hydroxyl or one chlorine group;

B is H, $COR_4$ or $SO_2R_5$, provided that when B is $COR_4$ or $SO_2R_5$; A is $COOR_3$ in which $R_3$ is other than H

or a cation, $CH_3$ or CN; W is O; and Y and Z are not alkylamino, hydroxyl, or hydroxyloweralkyl;
$R_4$ is $C_1$-$C_{11}$ alkyl, chloromethyl or phenyl optionally substituted with one chloro, one nitro or one methoxy group;
$R_5$ is $C_1$-$C_4$ alkyl or phenyl optionally substituted with one methyl group;
W is O or S;
$R_8$ is $C_1$-$C_4$-alkyl or phenyl;
Y and Z are each hydrogen, methyl;
$C_2$-$C_6$ straight or branched alkynyl optionally substituted with hydroxy;
$C_3$-$C_6$ cycloalkyl optionally interrupted by 1 oxygen, sulfur, amino, or $C_1$-$C_4$ alkylamino; oxiranyl optionally substituted by one or two $C_1$-$C_4$ alkyl;
$C_1$-$C_4$ alkyl substituted with one or more of the following groups:
$C_1$-$C_4$ alkoxy optionally substituted with phenyl, thienyl, furyl, cyclopropyl, tetrahydrofuryl, $C_1$-$C_4$ alkoxy, $C_1$-$C_4$ dialkylamino, $C_1$-$C_4$ alkylamino, $C_1$-$C_4$ trialkylammonium, or 1 to 3 halogens; with the proviso that when the substituent on the $C_1$-$C_4$ alkoxy group is $C_1$-$C_4$ alkoxy, $C_1$-$C_4$ alkylamino, $C_1$-$C_4$ dialkylamino, or $C_1$-$C_4$ trialkylammonium, the respective heteroatoms are separated by at least 2 carbon atoms;
$C_1$-$C_4$ alkenyloxy optionally substituted with 1 to 3 halogens;
$C_1$-$C_4$ alkynyloxy optionally substituted with 1 to 3 halogens;
$C_3$-$C_6$ cycloalkoxy;
phenyl thio;
$C_1$-$C_4$ alkylthio optionally substituted with phenyl or 1 to 3 halogens;
$C_1$-$C_4$ alkylsulfinyl optionally substituted with phenyl or 1 to 3 halogens;
$C_1$-$C_4$ alkylsulfonyl optionally substituted with phenyl or 1 to 3 halogens;
$C_1$-$C_4$ alkylamino optionally substituted on carbon by phenyl or 1 to 3 halogens;
cyano;
phenylamino, optionally substituted in the ring by 1 to 3 halogens, $C_1$-$C_4$ lower alkyl, or $C_1$-$C_4$ lower alkoxy;
phenoxy, optionally substituted in the ring by 1 to 3 halogens, $C_1$-$C_4$ lower alkyl, or $C_1$-$C_4$ lower alkoxy;
formyl;
amino optionally substituted with $C_1$-$C_4$ alkylsulfonyl and $C_1$-$C_2$ alkyl, amino, $C_1$-$C_4$ monoalkylamino, or $C_1$-$C_4$ dialkly amino;
Provided that at least one of Y and Z is:
$C_2$-$C_6$ straight or branched alkynyl optionally substituted with hydroxy;
$C_3$-$C_6$ cycloalkyl optionally interrupted by one oxygen, sulfur, amino, or $C_1$-$C_4$ alkylamino; oxiranyl optionally substituted by one or two $C_1$-$C_4$ alkyl;
$C_1$-$C_4$ alkyl substituted with one or more of the following groups:
$C_1$-$C_4$ alkoxy optionally substituted with phenyl, thienyl, furyl, cyclopropyl, tetrahydrofuryl, $C_1$-$C_4$ alkoxy, $C_1$-$C_4$ dialkylamino, $C_1$-$C_4$ alkylamino, $C_1$-$C_4$ trialkylammonium, or 1 to 3 halogens; with the proviso that when the substituent on the $C_1$-$C_4$ alkoxy group is $C_1$-$C_4$ alkoxy, $C_1$-$C_4$ alkylamino, $C_1$-$C_4$ dialkylamino, or $C_1$-$C_4$ trialkylammonium, the respective heteroatoms are separated by at least 2 carbon atoms;
$C_1$-$C_4$ alkenyloxy optionally substituted with 1 to 3 halogens;
$C_1$-$C_4$ alkynyl oxy optionally substituted with 1 to 3 halogens;
$C_3$-$C_6$ cycloalkoxy;
phenyl thio;
$C_1$-$C_4$ alkylthio optionally substituted with phenyl or 1 to 3 halogens;
$C_1$-$C_4$ alkylsulfinyl optionally substituted with phenyl or 1 to 3 halogens;
$C_1$-$C_4$ alkylsulfonyl optionally substituted with phenyl or 1 to 3 halogens;
$C_1$-$C_4$ alkylamino optionally substituted on carbon by phenyl or 1 to 3 halogens;
cyano;
phenylamino, optionally substituted in the ring by 1 to 3 halogens, $C_1$-$C_4$ lower alkyl, or $C_1$-$C_4$ lower alkoxy;
formyl; optionally substituted in the ring by 1 to 3 halogens, $C_1$-$C_4$ lower alkyl, or $C_1$-$C_4$ lower alkoxy;
formyl;
amino optionally substituted with $C_1$-$C_4$ alkylsulfonyl and $C_1$-$C_2$ alkyl, amino, $C_1$-$C_4$ monoalkylamino, or $C_1$-$C_4$ dialkyl amino;
Provided also that when Y is hydroxy, Z cannot be hydrogen;
The N-oxides thereof when W is O provided that $R_3$ cannot be unsaturated alkyl and Y and Z cannot be alkylthioalkyl, alkylaminoalkyl, dialkylaminoalkyl, trialkylammoniumalkyl, amino, 2(or 4)-pyridyloxy, alkoxyamino or unsaturated alkyl;
the optical isomers thereof when $R_1$ and $R_2$ are not the same;
the tautomers thereof; and

the acid addition salts thereof except when $R_3$ is a cation.

The present invention also relates to novel substituted pyrrolopyridine acetonitriles depicted by formula III, substituted pyrrolopyridine acetamides illustrated by formula IV, imidazopyrrolopyridinediones illustrated formulas V and VI, and carbamoyl nicotinic acids, esters and salts depicted by formula VII. These compounds have the structures illustrated below and are useful as intermediates for the preparation of the herbicidal nicotinic acids, esters and salts of formulas I and II.

Formula III, IV, V, VI and VII, compounds have the structures:

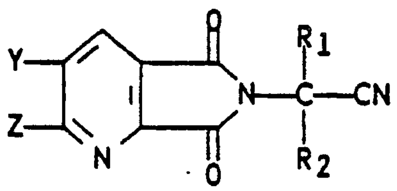

(III)

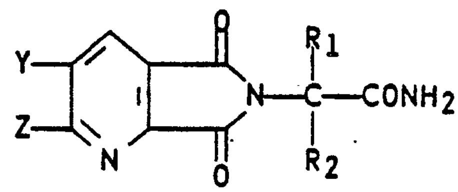

(IV)

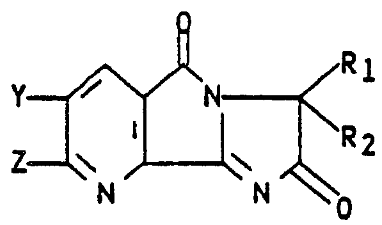

(V)

(VI)

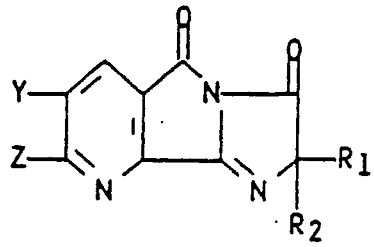

and

(VII)

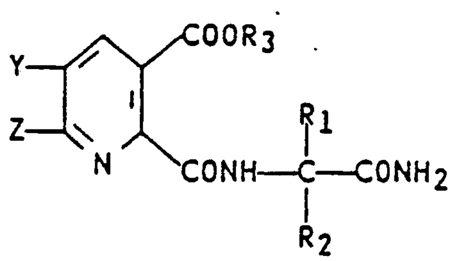

wherein $R_1$, $R_2$, $R_3$, Y and Z are as described above.

In accordance with this invention, 6-(4-isopropyl-4-methyl-5-oxo-2-imidazolin-2-yl)pyridine-3-carboxaldehyde is prepared by oxidation of 2-(5-hydroxymethyl-2-pyridyl)-4-isopropyl-4-methyl-2-imidazolin-5-one with manganese dioxide at an elevated temperature in the presence of an inert organic solvent such as a chlorinated hydrocarbon.

The thus prepared carboxaldehyde can then be converted to the desired 2-(5-alkenyl-2-pyridyl)-4-isopropyl-4-methyl-5-oxo-2-imidazolin-4-one by reaction of said aldehyde with the appropriate alkyl triphenylphosphonium halide in the presence of an alkali metal hydride, mineral oil and dimethylsulfoxide. Lithiation of this 5-alkenyl derivative, using methyl lithium and carbon dioxide in the presence of tetrahydrofuran, affords the corresponding, herbicidally active, 2-(4-isopropyl-4-methyl-5-oxo-2-imidazolin-2-yl)-5-alkenylnicotinic acid.

Esterification of the thus prepared nicotinic acid can then be achieved by reaction of said acid with ethereal diazoalkane, preferably in the presence of an inert organic solvent such as chlorinated hydrocarbon.

Preparation of the 5(and/or 6) substituted nicotinic acids wherein the 5(and/or 6) substitution is alkenyl, alkylcarbonyl, 2(or 3)-furyl or 2(or 3)-thienyl, can be accomplished from the appropriate 5(and/or 6)-substituted-2,3-dicarboxylate.

In this method the appropriately substituted 5(and/or 6) pyridine-2,3-dicarboxylate is converted to the corresponding acid by heating with a strong base, such as an alkali metal hydroxide, and then acidifying the reaction mixture with a strong mineral acid. Heating of the thus formed acid with acetic anhydride, preferably in the presence of pyridine and 1,2-dimethoxyethane, yields the 5(and/or 6)substituted pyridine-2,3-dicarboxylic anhydride. The 5(and/or 6) substituted pyridine-2,3-dicarboxylic anhydride is then reacted with an aminocarboxamide to yield an isomeric mixture of the 5(and/or 6)substituted carbamoylnicotinic acid and the 5(and/or 6)substituted carbamoylpicolinic acid.

This reaction is carried out using approximately equimolar amounts of the anhydride and the carboxamide, in the presence of an inert organic solvent such as a chlorinated hydrocarbon or a low-boiling ether such as THF, diethyl ether, or dimethoxyethane. When the reaction is essentially complete, the product mixture is heated with base to give a mixture of the 2-(4-isopropyl-4-methyl-5-oxo-2-imidazolin-2-yl) 5-(and/or 6) substituted nicotinic acid and the 3-(4-isopropyl-4-methyl-5-oxo-2-imidazolin-2-yl)-5-(and/or 6)-substituted picolinic acid. Conversion of this acid mixture to the corresponding esters can then be achieved by reaction of said mixture with acetic anhydride, preferably in the presence of an inert organic solvent such as toluene, xylene, benzene or the like. The reaction provides a mixture of the tricyclic diones of formulas V and VI which can be ring opened with an alkali metal alkoxide to yield a mixture of the 2-(4-isopropyl-4-methyl-5-oxo-2-imidazolin-2-yl) 5-(and/or 6)substituted nicotinate and the3-(4-isopropyl-4-methyl-5-oxo-2-imidazolin-2-yl) 5-(and/or 6) substituted picolinate.

Conversion of the alkyl 2-(4-isopropyl-4-methyl-5-oxo-2-imidazolin-2-yl) 5-(and/or 6)alkenyl nicotinates or the corresponding 5-(and/or 6)alkylthioalkyl or alkoxyalkylnicotinates to the corresponding acid can be achieved by reaction of the ester with alcoholic alkali metal hydroxide at an elevated temperature. After heating the reaction mixture is cooled and acidified with a strong mineral acid. The reaction is concentrated and then partitioned between a chlorinated hydrocarbon solvent and water.

Preparation of formula I, 5(and/or 6)haloalkyl 2-(2-imidazolin-2-yl)nicotinates can be achieved by reacting an alkyl 2-(4-isopropyl-4-methyl-5-oxo-2-imidazolin-2-yl)-5-alkylnicotinate with an N-halosuccinimide and benzoyl peroxide in the presence of an inert organic solvent such a chlorinated hydrocarbon. This reaction is generally conducted at an elevated temperature, preferably under a blanket of inert gas. The reaction yields the alkyl 2-(4-isopropyl-4-methyl-5-oxo-2-imidazolin-2-yl)-5(and/or 6)-(1-haloalkyl)nicotinate. If this reaction is conducted using an imidazolinyl function in which B is $COR_4$ as described above, the $COR_4$ function is retained during the reaction but can be removed from the product ester by reaction thereof with aqueous alcohol and a strong mineral acid. These reactions are exemplified by examples 9 and 10 described in detail below. The thus obtained alkyl 2-(4-isopropyl-4-methyl-5-oxo-2-imidazolin-2-yl)-5-(and/or 6)-(1-haloalkyl)nicotinate can then be used as the starting material for the preparation of the corresponding formula I 5(and/or 6)alkyl substituted with alkylcarbonyloxy 2-(2-imidazolin-2-yl)nicotinate. The reaction involves the treatment of the 5(and/or 6)-1-(haloalkyl)nicotinate referred to above, with an organic acid and an alkali metal salt of said organic acid.

Treatment of the thus prepared formula I 2-(2-imidazolin-2-yl)nicotinate, having a 5(and/or 6)alkyl substituent substituted with an alkylcarbonyloxy function, with an alkali metal alkoxide in a lower aliphatic alcohol provides the corresponding 5(and/or 6)hydroxyloweralkyl function on the pyridine ring of the 2-(2-imidazolin-2-yl)nicotinate.

Treatment of this 5-(and/or 6)hydroxy loweralkyl 2-(2-imidazolin-2-yl) nicotinate with pyridinium dichromate and pyridinium trifluoroacetate in the presence of an inert organic solvent such as a chlorinated hydrocarbon, then yields the corresponding 5(and/or 6)alkylcarbonyl 2-(2-imidazolin-2-yl)nicotinate.

To obtain the formula I, 5(and/or 6)alkylthioalkyl substituted 2-(2-imidazolin-2-yl)nicotinates of this invention, a formula I 5(and/or 6)haloalkyl 2-(2-imidazolin-2-yl)nicotinate is dissolved in absolute methanol, treated with a 50% oil dispersion of alkali metal hydride and then with an alkyl mercaptan. These additions are generally made while maintaining the temperature of the mixture below about -5 °C. Thereafter, the mixture is acidified with acetic acid to pH 4 and then concentrated in vacuo. The residue can be dissolved in methylene chloride, washed with aqueous sodium bicarbonate, reconcentrated in vacuo and the product recovered.

Additional methods for the preparation of the compounds of this invention are described in the examples below.

The formula I compounds of this invention are effective herbicidal agents useful for the control of a variety of monocotyledonous and dicotyledonous plants. These compounds are herbicidally effective for controlling weeds indigenous to both dry land and wet land areas. They are also useful as aquatic herbicides and are effective for controlling the above-said plants when applied to the foliage thereof or to the soil or water containing seeds or other propagating organs of said plants such as tubers, rhizomes or stolons, at rates of from about 0.016 to 8.0 kg/ha.

The formula I and formula II compounds of this invention can be formulated as wettable powders, flowable concentrates, granular formulations and the like.

Wettable powders can be prepared by grinding together about 20% to 45% by weight of a finely divided carrier such as kaolin, bentonite, diatomaceous earth, attapulgite, or the like, 45% to 80% by weight of the active compound, 2% to 5% by weight of a dispersing agent such as sodium lignosulfonate, and 2% to 5% by weight of a nonionic surfactant, such as octylphenoxy polyethoxy ethanol, nonylphenoxy polyethoxy ethanol or the like.

A typical flowable liquid can be prepared by admixing about 40% by weight of the active ingredient with about 2% by weight of a gelling agent such as bentonite, 3% by weight of a dispersing agent such as sodium lignosulfonate, 1% by weight of polyethylene glycol and 54% by weight of water.

A typical emulsifiable concentrate can be prepared by dissolving about 5% to 25% by weight of the active ingredient in about 65% to 90% by weight of N-methylpyrrolidone, isophorone, butyl cellosolve, methylacetate or the like and dispersing therein about 5% to 10% by weight of a nonionic surfactant such as an alkylphenoxy polyethoxy alcohol. This concentrate is dispersed in water for application as a liquid spray.

When the compounds of the invention are to be used as herbicides where soil treatments are involved, the compounds may be prepared and applied as granular products. Preparation of the granular product can

be achieved by dissolving the active compound in a solvent such as methylene chloride, N-methylpyrrolidone or the like and spraying the thus prepared solution on a granular carrier such as corncob grits, sand, attapulgite, kaolin or the like.

The granular product thus prepared generally comprises about 3% to 20% by weight of the active ingredient and about 97% to 80% by weight of the granular carrier.

The following examples illustrate the invention.

EXAMPLE 1

Preparation of 5-(3-hydroxy-3-methyl-1-butynyl)-2-(4-isopropyl-4-methyl-5-oxo-2-imidazolin-2-yl)nicotinic acid, methyl ester

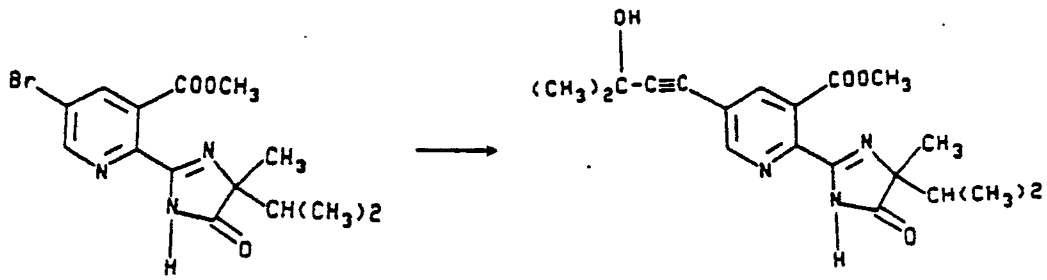

A stirred mixture of 12.6 g of 5-bromo-2-(4-isopropyl-4-methyl-5-oxo-2-imidazolin-2-yl)nicotinic acid, methyl ester, 3.4g of 3-hydroxy-3-methyl-1-butyne, 0.02 g of bis(triphenylphosphine)palladium(II)-chloride, 0.04 g of triphenylphosphine, and 0.02 g of cuprous iodide in 300 mL of dry triethylamine is heated at reflux for 72 hours. The reaction is then filtered hot through celite, and the filtering agent is washed with methylene chloride. The combined filtrates were concentrated in vacuo, and the residue is partitioned between dilute aqueous hydrochloric acid and methylene chloride. The organic phase is washed with brine, dried, concentrated in vacuo, and the residue is chromatographed on silica gel using ether-methylene chloride mixtures to afford the desired product, mp 142-145°.

EXAMPLE 2

Preparation of 5-ethynyl-2-(4-isopropyl-4-methyl-5-oxo-2-imidazolin-2-yl)nicotinic acid

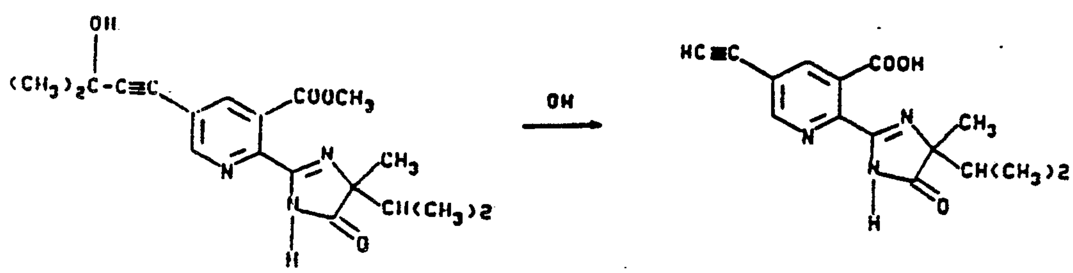


A solution of 1.5g of 5-(3-hydroxy-3-methyl-1-butynyl)-2-(4-isopropyl-4-methyl-5-oxo-2-imidazolin-2-yl)-nicotinic acid, methyl ester, and 0.185g of sodium hydroxide in 12 mL water is heated at reflux for 2 1/2 hours, then stirred at room temperature overnight. The reaction is then cooled to 10°, acidified to pH 3 with concentrated hydrochloric acid and extracted with methylene chloride. The organic phase is dried and

concentrated in vacuo to afford the crude, desired product. An analytical sample, purified via its methyl ester, had mp 190-195°.

## EXAMPLE 3

Preparation of 5-(1-propynyl)-pyridine-2,3-dicarboxylic acid, diethyl ester

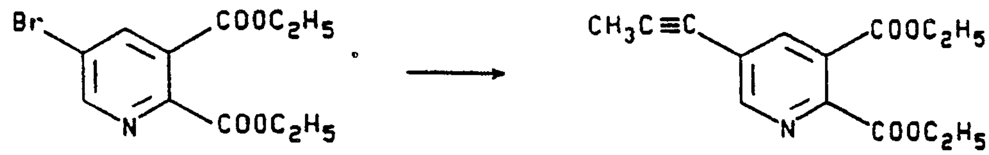

A stirred mixture of 3g of 5-bromo-pyridine-2,3-dicarboxylic acid, diethyl ester, 0.1g cuprous iodide and 0.7g bis(triphenylphosphine)palladium(II)chloride in 40 mL dimethyl sulfoxide and 12 mL triethylamine is cooled to 10°, and 10 mL of liquid propyne is added. The reaction is stirred at room temperature thereafter for 16 hours, then is poured onto water and extracted with methylene chloride. The organic phase is washed with dilute hydrochloric acid, then water, then brine, and dried. Concentration in vacuo, and chromatography of the residue on silica gel using ether-hexane mixture affords the desired product as an oil.

## EXAMPLE 4

Preparation of 2-(4-isopropyl-4-methyl-5-oxo-2-imidazolin-2-yl)-5-[(phenylsulfinyl)methyl]nicotinic acid, methyl ester

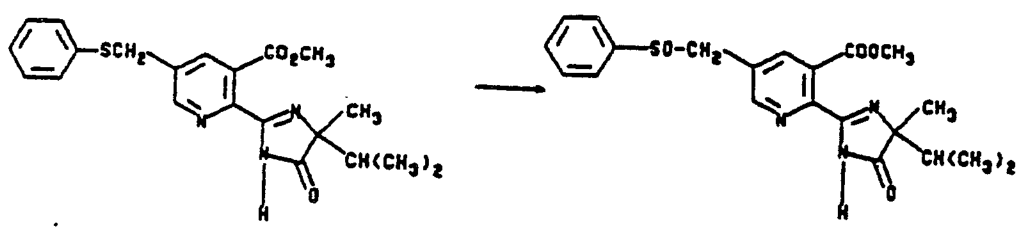

To a solution of 1.0 g of 2-(4-isopropyl-4-methyl-5-oxo-2-imidazolin-2-yl)-5-[(phenylthio)methyl]-nicotinic acid, methyl ester, in 20 mL methylene chloride is added 0.2 g sodium bicarbonate. The stirred mixture is cooled to -78°C under nitrogen, and a solution of 0.51 g meta-chloroperoxybenzoic acid in 20 mL methylene chloride is added in one portion. The reaction mixture is stirred at -78°C for 30 minutes, allowed to warm to room temperature over 1 hour, then stirred overnight. The reaction is poured onto 5% aqueous sodium bisulfite, and the organic phase is separated, washed with aqueous sodium bicarbonate, dried over magnesium sulfate, and concentrated in vacuo. The residue is chromatographed on silica gel using hexane-ethylacetate mixtures to afford the desired product, mp 132-136°.

By optionally using an excess of meta-chloroperbenzoic acid in the above procedure, optionally hydrolyzing the ester subsequent to the oxidation, and using the appropriate nicotinate precursor, one obtains the following:

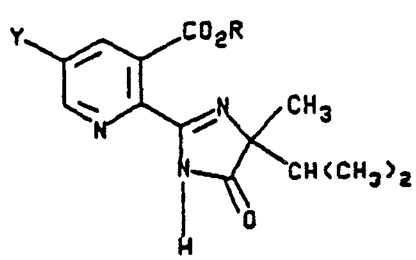

| Y | R | mp°C |
|---|---|---|
| $C_6H_5SO_2CH_2$- | $CH_3$ | 68 - 77 |
| $CH_3SOCH_2$- | H | 190 - 192 |
| $CH_3SO_2CH_2$- | H | oil |
| $(CH_3)_2CHSOCH_2$- | H | solid |
| $C_6H_5CH_2SO_2CH_2$- | H | 140 - 143 |

EXAMPLE 5

Preparation of 2-(1-benzoyl-4-isopropyl-4-methyl-5-oxo-2-imidazolin-2yl)-5-[(phenylsulfonyl)methyl]nicotinic acid, methyl ester

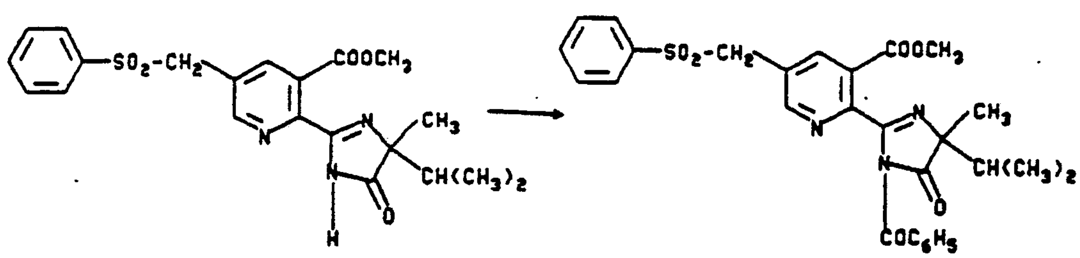

A solution of 5.9g of 2-(4-isopropyl-4-methyl-5-oxo-2-imidazolin-2-yl)-5-[(phenylsulfonyl)methyl]-nicotinic acid, methyl ester, and 0.3g p-dimethylaminopyridine in 50 mL pyridine is stirred at room temperature and 1.75 mL benzoyl chloride is added in one portion. After stirring overnight, the reaction is concentrated in vacuo and the residue is partitioned between methylene chloride and aqueous sodium bicarbonate. The aqueous phase is further extracted with methylene chloride, and the combined organic phases are dried and concentrated in vacuo. Chromatography of the residue on silica gel using hexane-ethyl acetate mixtures affords the desired product, mp 162-166°.

Using essentially the same procedure, and substituting the appropriate pyridine precursor, one obtains the following:

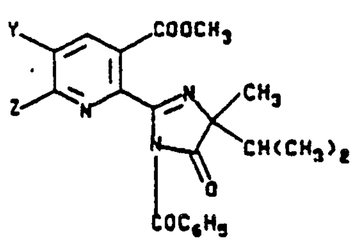

| Y | Z | mp |
|---|---|---|
| $CH_3OCH_2$ | H | gum |
| $CH_3$ | H | gum |
| $C_2H_5$ | H | gum |

EXAMPLE 6

Preparation of 5-(cyanomethyl)-2-(4-isopropyl-4-methyl-5-oxo-2-imidazolin-2-yl)nicotinic acid

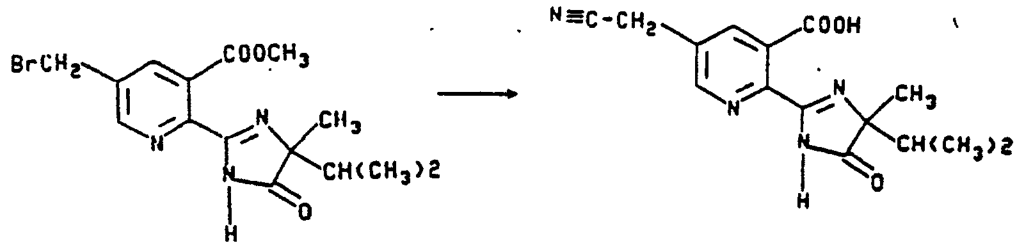

To a solution of 1.0 g of 2-(1-benzoyl-4-isopropyl-4-methyl-5-oxo-2-imidazolin-2-yl)-5-bromomethylnicotinic acid, methyl ester, in 60 mL ethanol is added 0.24 g sodium cyanide, followed by 10 cc water. The resulting solution is stirred at room temperature for 24 hours and concentrated in vacuo. The residue is partitioned between methylene chloride and water. The aqueous phase is acidified to pH 3 and extracted with methylene chloride, and the combined organic phases are dried and concentrated in vacuo. Chromatography of the residue on silica gel using methylene chloride-methanol mixtures affords the desired product, mp 196-200° (dec).

## EXAMPLE 7

Preparation of 5-cyclopropyl-2-(4-isopropyl-4-methyl-5-oxo-2-imidazolin-2yl)nicotinic acid

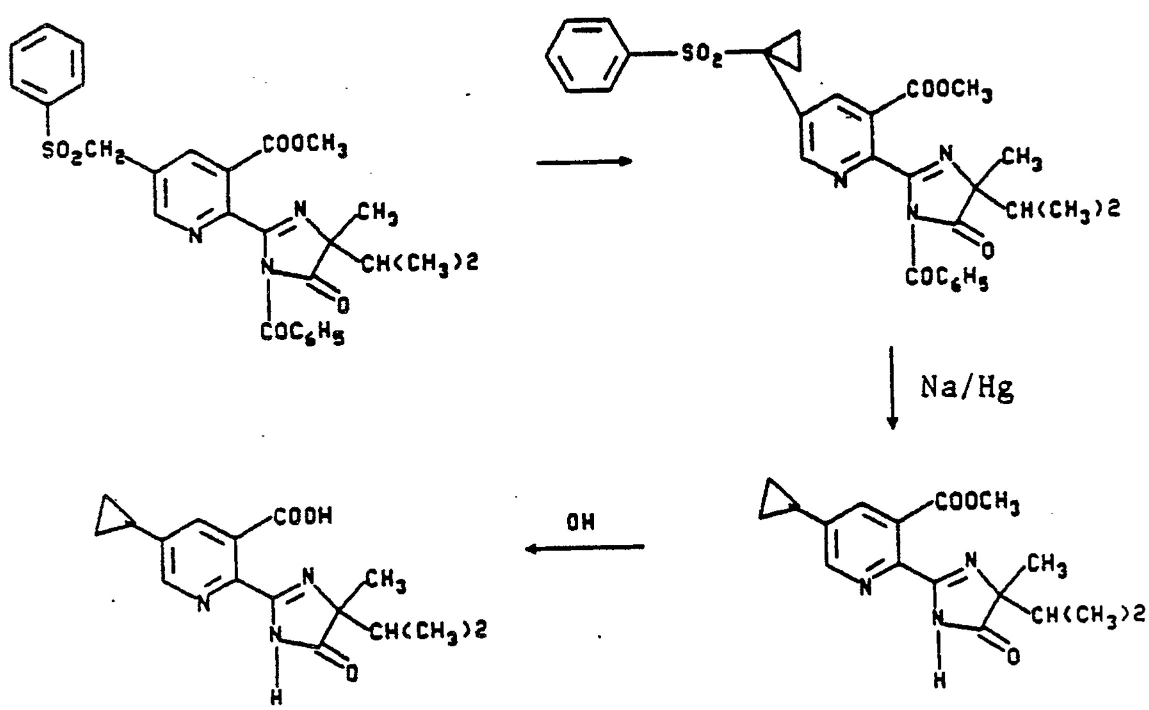


To a solution of 2.0g potassium tert-butoxide in 10 mL dimethylformamide, stirred at -10° C, is added a solution of 2.38g of 2-(1-benzoyl-4-isopropyl-4-methyl-5-oxo-2-imidazolin-2-yl)-5-[(phenylsulfonyl)methyl]-nicotinic acid, methyl ester, in 15 mL dimethylformamide. After stirring at -10° for an additional 20 minutes, 2.25g of (2-chloroethyl)dimethylsulfonium iodide is added, and the reaction is stirred at -10° for 1.5 hours, then at 0° for 1 hour. The reaction mixture is quenched with 2g of ammonium chloride and concentrated in vacuo. The residue is partitioned between methylene chloride and aqueous ammonium chloride, and the

organic phase is dried and concentrated in vacuo. The residue is chromatographed on silica gel using hexane-ethyl acetate mixtures to afford a mixture of benzoylated and non-benzoylated 2-(4-isopropyl-4-methyl-5-oxo-2-imidazolin-2-yl)-5-[(1-phenylsulfonyl)-1-cyclopropyl]nicotinic acid, methyl esters.

This mixture is re-benzoylated according to Example 40 to afford 2-(1-benzoyl-4-isopropyl-4-methyl-5-oxo-2-imidazolin-2-yl)-5-[(1-phenylsulfonyl)-1-cyclopropyl]nicotinic acid, methyl ester as a gum.

To a solution of 0.3g of this material in 3 mL methanol containing 0.69g disodiumhydrogen phosphate is added at 0°. 1.84g of solid 6% sodium-mercury amalgam. The reaction mixture is stirred at 0° for 6.5 hours, then diluted with 100 mL aqueous ammonium chloride and extracted with methylene chloride. The combined extracts were dried and concentrated in vacuo to afford crude 5-cyclopropyl-2-(4-isopropyl-4-methyl-5-oxo-2-imidazolin-2yl)nicotinic acid, methyl ester. Hydrolysis of this material according to Example 9 and chromatography of the residue on silica gel using methylene chloride-methanol mixtures affords the desired product, mp 157-163°.

## EXAMPLE 8

Preparation of 5-(dimethoxymethyl)-2-(4-isopropyl-4-methyl-5-oxo-2-imidazolin-2-yl)nicotinic acid, methyl ester, and 2-(4-isopropyl-4-methyl-5-oxo-2-imidazolin-2-yl)-6-methoxy-5-(methoxymethyl)nicotinic acid, methyl ester

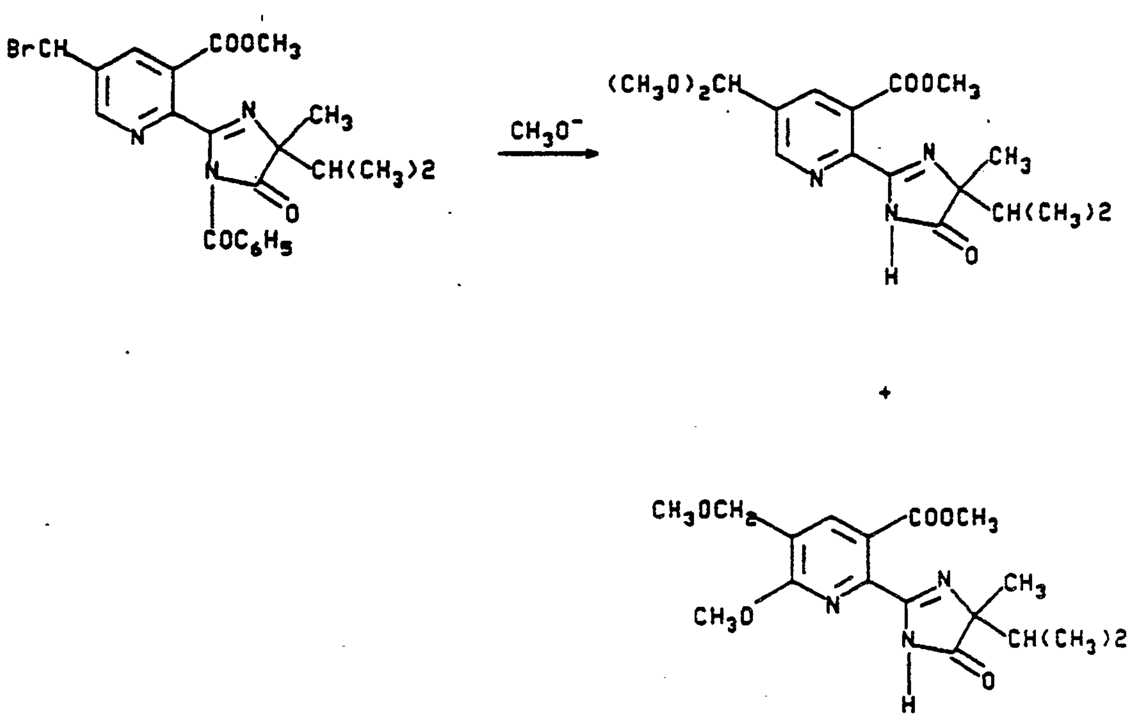


To a solution of 0.50g of 5-(dibromomethyl)-2-(1-benzoyl-4-isopropyl-4-methyl-5-oxo-2-imidazolin-2-yl)-nicotinic acid, methyl ester, in 10 mL methanol is added 0.20g sodium methoxide, and the reaction is heated at reflux for 5 hours. The reaction is then cooled, concentrated in vacuo, and the residue is digested in aqueous ammonium chloride and extracted with methylene chloride. The combined extracts are dried, concentrated in vacuo, and the residue is chromatographed on silica gel using hexane-ethyl acetate mixtures to afford 0.15g of 5-(dimethoxymethyl)-2-(4-isopropyl-4-methyl-5-oxo-2-imidazolin-2-yl)nicotinic acid, methyl ester, as a colorless oil, and 0.07g of 2-(4-isopropyl-4-methyl-5-oxo-2-imidazolin-2-yl)-6-methoxy-5-(methoxymethyl)nicotinic acid, methyl ester as a white solid.

## EXAMPLE 9

Preparation of 5-chloromethyl-2,3-pyridinedicarboxylic acid anhydride

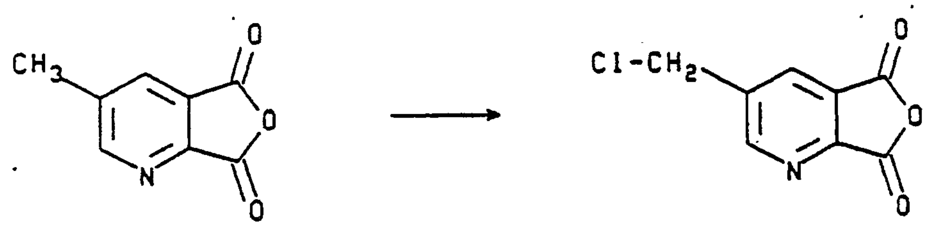

A solution of 75.0 g (0.41 m) of 5-methyl-2,3-pyridinedicarboxylic acid in 500 mL of 1,2-dimethoxyethane is treated with 125 g acetic anhydride (1.2 m) and 81 g pyridine (1.02 m). The resulting solution is stirred for 18 hours at room temperature. The solution is stripped in vacuo to leave 70.3 g (100%) of 5-methyl-2,3-pyridinedicarboxylic acid anhydride. The anhydride is dissolved in 500 mL $CCl_4$ and is treated with 1g AIBN and 240 g (150 mL, 1.8 m) $SO_2Cl_2$ at room temperature. The solution is heated to reflux for 2 hours. An additional 1.0 g AIBN and 80 g (50 mL 0.6 m) $SO_2Cl_2$ are added and refluxing continued for an additional 2 hours. The solution, with some suspended solids, is cooled to 15° in an icebath and filtered to leave 56.0 g (69%) of a yellow solid: 5-chloromethyl-2,3-pyridinedicarboxylic anhydride (78% by nmr) mp (softens at 90° C) 141-145° C. Alternatively, the product can be hydrolyzed to give 5-chloromethyl-2,3-pyridinedicarboxylic acid, mp 170-172°, from which the anhydride can be reformed by standard methods (see Example 6).

## EXAMPLE 10

Preparation of 2-[(1-carbamoyl-1,2-dimethylpropyl)carbamoyl]-5-(chloromethyl)nicotinic acid, triethylamine salt

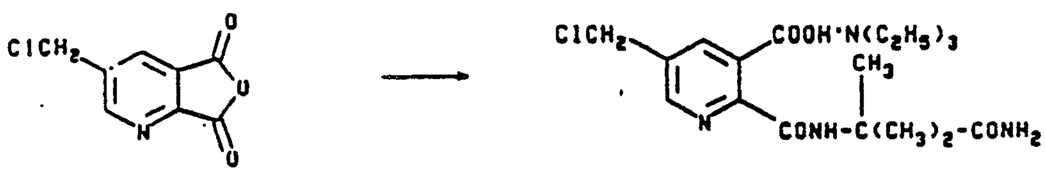

A solution of 5-(chloromethyl)-2,3-pyridinedicarboxylic acid anhydride [72.0 g (78% real) 0.3 m] in 300 mL $CH_3CN$ is cooled in an ice-salt bath to 0°. A solution of 39.0 g (0.3 m) α-methylvaleramide and 33.4 g (0.33 m) triethylamine in 100 mL $CH_3CN$ is added with stirring over 30 minutes with temperature ≤ 10°. The resulting suspension is stirred for an additional hour and filtered. The white solid is washed with 100 mL $Et_2O$ and is dried. There is obtained 83.2 g (66%) of 2-[(1-carbamoyl-1,2-dimethylpropyl)carbamoyl]-5-chloromethylnicotinic acid, triethylamine salt, mp (shrinks at 148°) 159-160°.

## EXAMPLE 11

Preparation of 5-methoxymethyl-2-(4-isopropyl-4-methyl-5-oxo-2-imidazolin-2yl)nicotinic acid, triethylamine salt

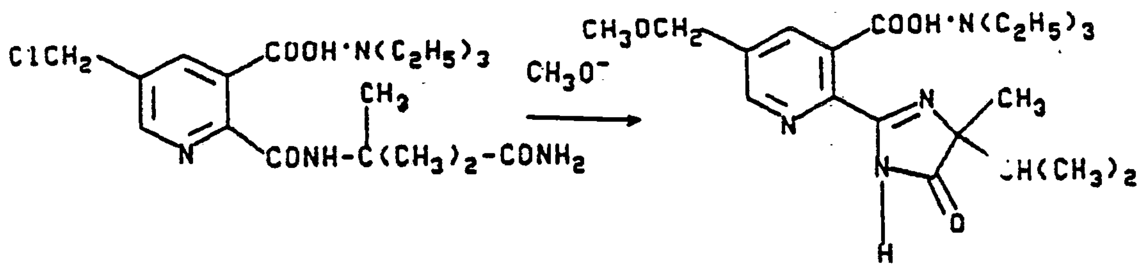


A solution of 41.0 g (0.096 m) of 2-[(1-carbamoyl-1,2-dimethylpropyl)carbamoyl]-5-chloromethylnicotinic acid, triethylamine salt in 250 mL absolute methanol is treated with 26.0 g (0.48 m) solid sodium methoxide. The resulting suspension is heated to reflux and held there for 2 hours. The reaction mixture is cooled to room temperature, and the pH is adjusted to 4 with concentrated hydrochloric acid and is filtered. The filtrate is evaporated in vacuo to leave 37.7 g (96%) of 2-(4-isopropyl-4-methyl-5-oxo-2-imidazolin-2-yl)-5-methoxymethylnicotinic acid, triethylamine salt as a yellow solid, mp. 78-80 °C.

Using essentially the same procedure and substituting the appropriate nucleophile, and acidifying to $pH_2$ in the final workup soas to obtain the free acid directly, the following are obtained:

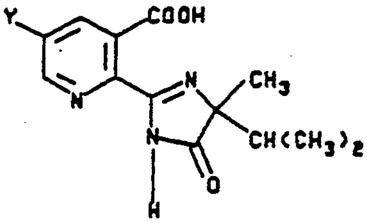

| Y | mp |
|---|---|
| $CH_2=CHCH_2OCH_2$ | 147.0 - 149.0° |
| $CH_3-CH(C_2H_5)OCH_2$ | 142.0 - 144.0° |
| $CH\equiv C-CH_2OCH_2$ | 125.0 - 128.0° |
| $CH_3(CH_2)_3OCH_2$ | 139.0 - 141.0° |
| $CH_3CH_2OCH_2$ | 160.0 - 162.0° |
| $CH_3OCH_2CH_2OCH_2$ | 115.0 - 116.0° |
| $CF_3CH_2OCH_2$ | 201.0 - 203.0° |
| $CH_3(CH_2)_2OCH_2$ | 159.0 - 160.5° |
| 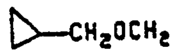 | 141.5 - 143.0° |
| $(CH_3)_2CHOCH_2$ | 167.0 - 169.0° |
| $CH_2=CH(CH_2)_2OCH_2$ | 146.0 - 148.0° |
| $FCH_2CH_2OCH_2$ | 146.0 - 148.0° |
| 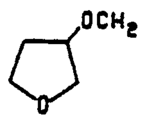 | 129.0 - 132.0° |
| 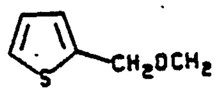 | - |
| $Cl_3CCH_2OCH_2$ | 186.0 - 188.0° |
| 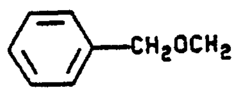 | 168.0 - 169.0° |
| $(CH_3)_2CHCH_2OCH_2$ | 172.0 - 174.0° |
| 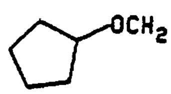 | 142.0 - 143.0° |
| $CH_3C\equiv CCH_2OCH_2$ | 144.5 - 146.0° |
| 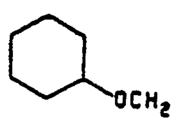 | 153.5 - 154.5° |
| 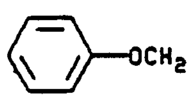 | 208.5 - 209.5° |

EXAMPLE 12

Preparation of 2-(4-isopropyl-4-methyl-5-oxo-2-imidazolin-2yl)-5-(methoxymethyl)nicotinic acid

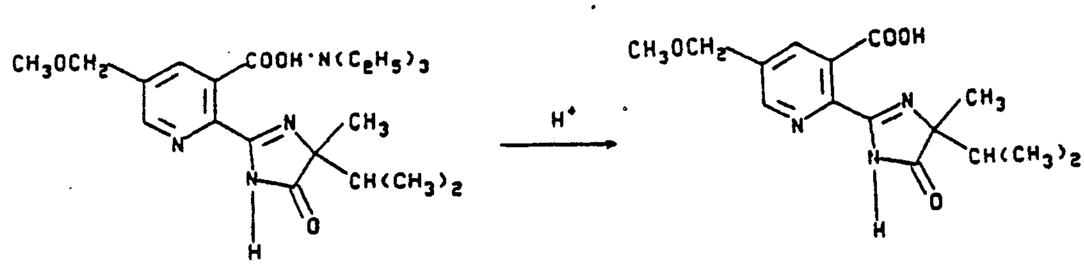


A solution of 2-(4-isopropyl-4-methyl-5-oxo-2-imidazolin-2yl)-5-(methoxylmethyl)nicotinic acid, triethylamine salt (37.7g, 0.09m) in 100 mL $H_2O$ is cooled in an ice bath and the pH adjusted to pH 1 to 2 with concentrated hydrochloric acid. The resultant white solid is filtered to give 22.5g (82%) of 2-(4-isopropyl-4-methyl-5-oxo-2-imidazolin-2yl)-5-(methoxymethyl)nicotinic acid, mp 158-160° C.

EXAMPLE 13

Preparation of 5-(dimethylaminomethyl)-2-(4-isopropyl-4-methyl-5-oxo-2-imidazolin-2-yl)nicotinic acid

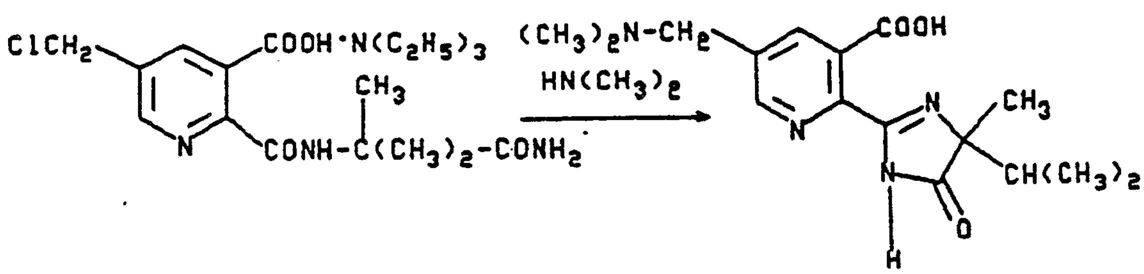


The triethylamine salt of 2-[(1-carbamoyl-1,2-dimethylpropyl)carbamoyl]-5-(chloromethyl)nicotinic acid (5.0g, 0.012 mole) is dissolved in 40% aqueous dimethylamine (13.5 mL, 0.120 mole) stirred at 30° for 2 hours, and concentrated *in vacuo* to give 5.1g of a yellow oil. A portion of this oil (3.94g, 0.010 mole) is dissolved in 10 mL 5N NaOH, stirred at 60° for 4 hours, cooled to room temperature, treated with 75 mL water, and acidified to pH 4 with concentrated hydrochloric acid. The water is removed *in vacuo*; the residue is taken up in ethanol, filtered, and concentrated *in vacuo*. The procedure is repeated; the final residue is dissolved in 50 mL water, and the solution is washed with methylene chloride and evaporated to dryness to give the product as a white solid, 0.34g, mp 186-190°.

Using essentially the same procedure, and substituting the appropriate nucleophile, the following are obtained:

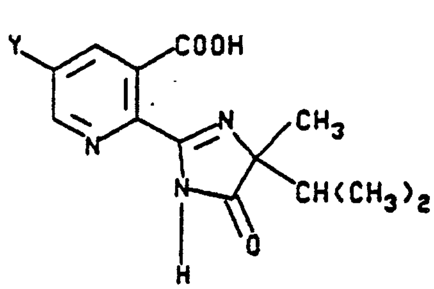

| Y | mp |
|---|---|
| 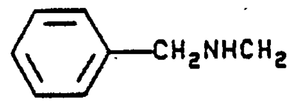 | 213.0 - 215.0° |
| $(CH_3)_2CHSCH_2$ | 145.0 - 147.0° |
| 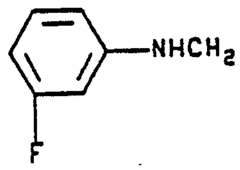 | 196.0 - 198.0° |
| 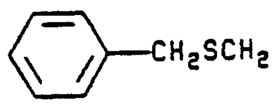 | 123.0 - 125.0° |

EXAMPLE 14

Preparation of 5-(1-methoxyethyl)-2-(4-isopropyl-4-methyl-5-oxo-2-imidazolin-2yl)nicotinic acid, methyl ester

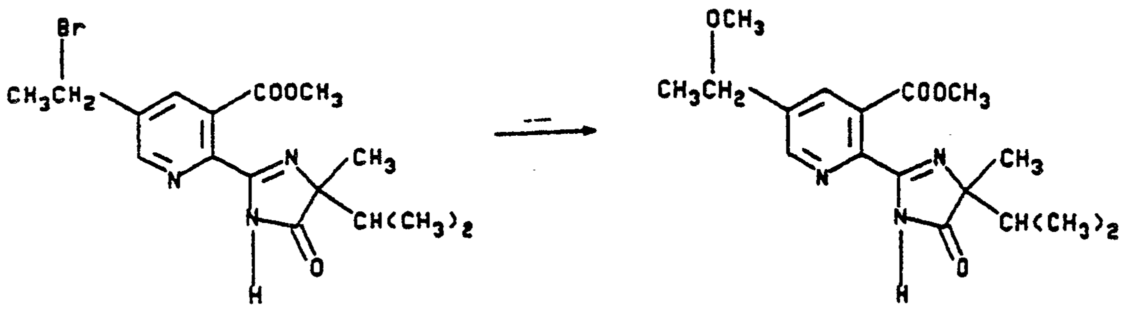

A solution of 5-(1-bromoethyl)-2-(4-isopropyl-4-methyl-5-oxo-2-imidazolin-2yl)nicotinic acid, methyl ester (9.2g, 0.024 mole) in absolute methanol (400 mL) is heated in a sealed pressure vessel at 120-140° for 18 hours. The solution is cooled and then stirred with $NaHCO_3$ (2.0g, 0.024 mole) for 1 hour. The mixture is filtered, and the filtrate is concentrated to leave a gum which is chromatographed twice on silica with ether as eluant to afford 1.52g of the product, a glass, which is dissolved in $CCl_4$. The solution is filtered to remove impurities and concentrated to leave the desired product, a glass, which still contained some $CCl_4$.

EXAMPLE 15

Preparation of diethyl 5-(1-hydroxyethyl)-6-methylpyridine-2,3-dicarboxylate

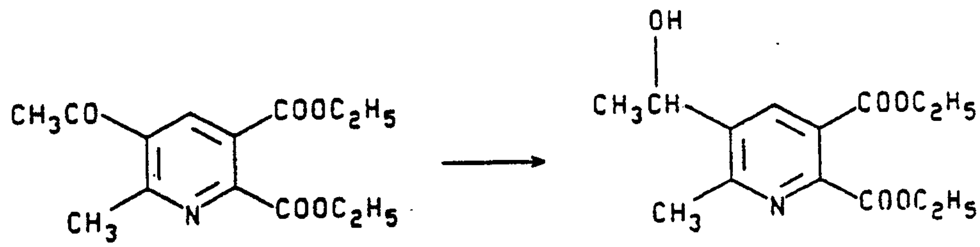

A solution of the starting material (55.8g, 0.20 mole) in absolute ethanol (140 mL) is added slowly to a cold (8°) solution of $NaBH_4$ (2.91g, 0.0767 mole) in absolute ethanol (200 mL). The cooling bath is then removed, and the solution is kept at 22° for 16 hours. The solution is then cooled to 8° and acidified to pH 2 with concentrated hydrochloric acid (ca. 8 mL). After a short time, the solution is treated with water (30 mL) and $Na_2CO_3$ (20g, 0.2 mole) and then filtered. The filtrate is concentrated to an oil which is dissolved in ether. The dried ($MgSO_4$) solution is in turn concentrated to leave the product, 57.2g of an amber oil, (98.5% purity).

EXAMPLE 16

Preparation of diethyl 5-(1-bromoethyl)-6-methyl-2,3-pyridinedicarboxylate

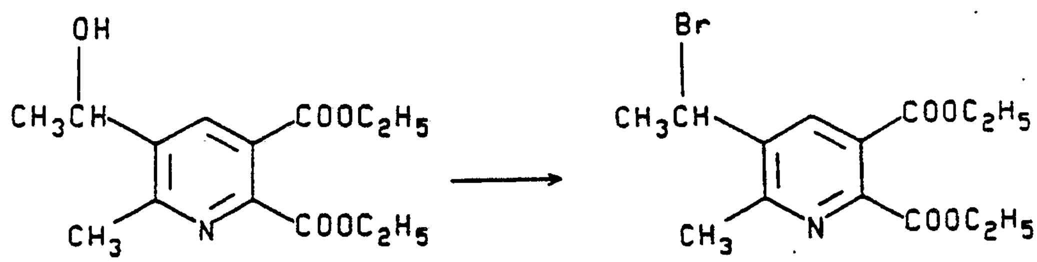

Phosphorous tribromide (7.0 mL, 0.074 mole) is added dropwise over 3 minutes to a solution of the 5-(1-hydroxyethyl)-6-methyl diester (52.0g, 0.185 mole) in dry dimethoxyethane (500 mL). The temperature rises from 22° to 33°. The mixture is stirred for 21 hours at 22° and then is basified to pH 6 by the addition of water (50 mL) and $NaHCO_3$ (50g). The organic layer is separated, washed with two 30 mL portions of aqueous $NaHCO_3$ solution, dried ($MgSO_4$), and concentrated to leave the 5-(1-bromoethyl)-6-methyl diester, a yellow oil, wt. 54.3g, (91.4% purity).

EXAMPLE 17

Preparation of diethyl 5-(1-methoxymethyl)-6-methyl-2,3-pyridinedicarboxylate

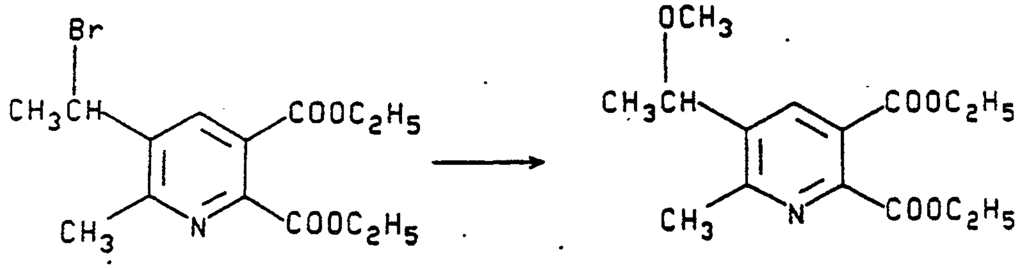

A solution of the 5-(1-bromoethyl)diester (51g, 0.148 mole) in methanol (500 mL) is heated at 120° in a sealed vessel for 28 hours. After cooling to room temperature, $NaHCO_3$ (12.5g, 0.15 mole) is added; the mixture is stirred, filtered, and the filtrate is concentrated in vacuo. The residue is dissolved in ether, treated with water and solid $NaHCO_3$ (12.5g). The mixture is stirred, and the phases are separated. The aqueous layer is extracted with four portions of ether; the organic phases are combined and washed with aqueous $NaHCO_3$ solution, dried ($MgSO_4$) and concentrated to leave 11.1g of the product as a dark oil.

## EXAMPLE 18

Preparation of 5-acetyl-6-methylpyridinedicarboxylic acid diethyl ester, oxime

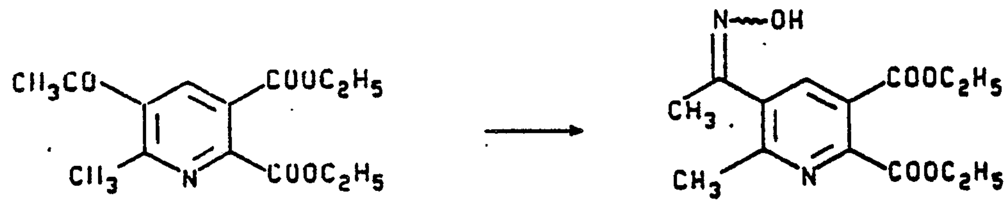

The starting material, 5-acetyl-6-methylpyridinediethyl ester, (59.5g, 0.213 mol) is dissolved in 590 mL of absolute ethanol. Hydroxylamine hydrochloride (16.3g, 1.1 equivalents) and sodium acetate (19.2g, 1.1 equivalents) are added, and the mixture is mechanically stirred for 24 hours. The precipitate is filtered, and the filter cake is washed with 100 mL of absolute ethanol. The filtrates are combined and concentrated in vacuo to yield 62.0g of a yellow oil.

Using essentially the same procedure and the appropriate pyridine precursor, one obtains the following:

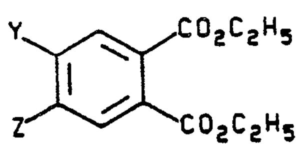

| Y | Z | mp |
|---|---|---|
| 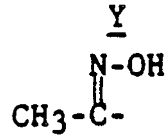 | H | oil |

## EXAMPLE 19

Preparation of 5-acetamido-pyridine-2,3-dicarboxylic acid, diethyl ester

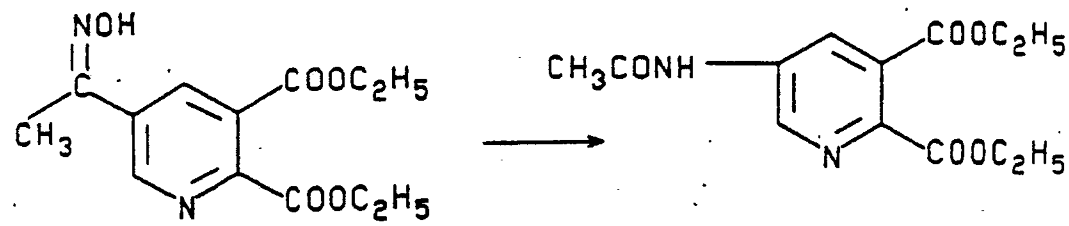

A stirred solution of 14g of 5-acetylpyridine-2,3-dicarboxylic acid, diethyl ester, oxime, in 350 mL of dry tetrahydrofuran is treated with 15.6g of phosphorous pentachloride in portions over a 20-minute period. The resulting dark reaction mixture is stirred at room temperature for 4 days, cooled in an ice bath, and quenched by the dropwise addition of 125 mL of saturated aqueous sodium bicarbonate. The aqueous phase is extracted with ethyl acetate, and the combined organic phases are washed with brine, dried,and concentrated in vacuo. The residue is dissolved in methylene chloride, washed with saturated bicarbonate, followed by water. Drying and concentration in vacuo affords a gum which is chromatographed on silica gel using ether-ethyl acetate mixtures to afford the desired product, mp 108-110°.

Using essentially the same procedure and substituting the appropriate pyridine precursor, one obtains the following:

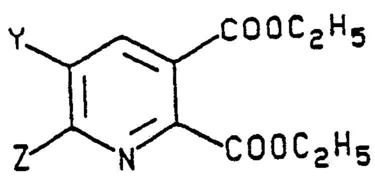

| Y | Z | mp°C |
|---|---|---|
| $CH_3CONH-$ | $CH_3-$ | 107 - 108 |

EXAMPLE 20

Preparation of 5-formyl-2-(4-isopropyl-4-methyl-5-oxo-2-imidazolin-2-yl)nicotinic acid, methyl ester

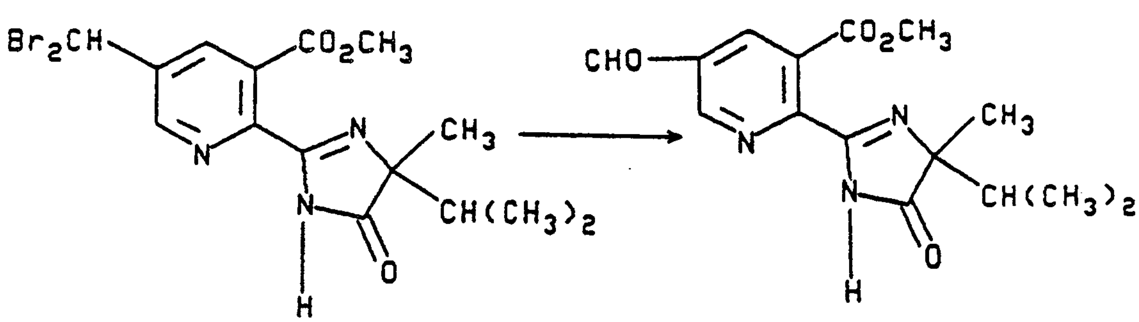

A stirred solution of 1.38 g of 2-(1-benzoyl-4-isopropyl-4-methyl-5-oxo-2-imidazolin-2-yl)-5-(dibromomethyl)nicotinic acid, methyl ester, in 35 mL tetrahydrofuran is cooled to -78° and treated with a mixture of 0.3 mL of N-ethylethylenediamine, 10 mL tetrahydrofuran, and 0.8 mL of triethylamine. After warming to room temperature, the reaction solution is treated with 0.3 mL of n-ethylethylenediamine, stirred for 2 hours, heated at reflux for 24 hours, and concentrated in vacuo. The residue is partitioned between methylene chloride and water; the aqueous phase is further extracted with methylene chloride, and the combined organic phases are dried and concentrated in vacuo. Chromatography of the residue on silica gel

using hexane-ethyl acetate mixtures affords the desired product, mp 113-117°.

## EXAMPLE 21

Preparation of 5-carboxy-6-(4-isopropyl-4-methyl-5-oxo-2-imidazolin-2-yl)-2-methyl-3-pyridinecarbamic acid, dimethyl ester

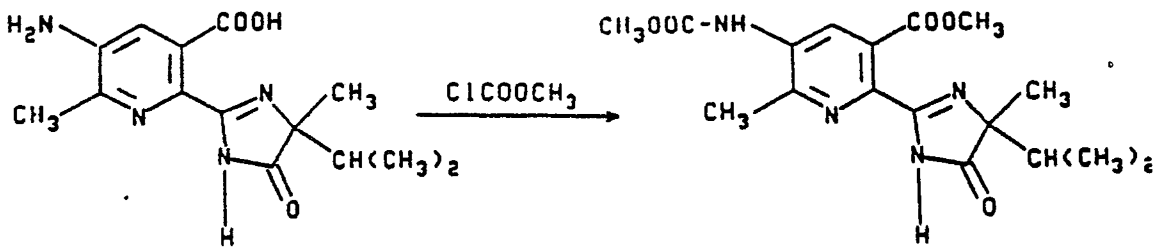


To a stirred solution of 5-amino-6-methyl-2-(4-isopropyl-4-methyl-5-oxo-2-imidazolin-2yl)nicotinic acid (5.0 g, 17.2 mmole) and pyridine (2.8 mL, 34.4 mmole) in 50 mL of dry dimethoxyethane is added methylchloroformate (2.7 mL, 34.4 mmole). The reaction is heated at reflux for 3 hours, then held at 50° for 16 hours. After cooling to room temperature, the reaction mixture is treated with 15 mL of methanol, basified to pH 7-8 with sodium methoxide, stirred at room temperature for 16 hours, and concentrated in vacuo. The residue is chromatographed using heptane-ethyl acetate mixtures to provide the product in 28% yield as a white solid, 1.75 g, mp 184-186°.

## EXAMPLE 22

Preparation of 2-chloro-3-(2-chloroethyl)-5,8-dimethoxyquinoline

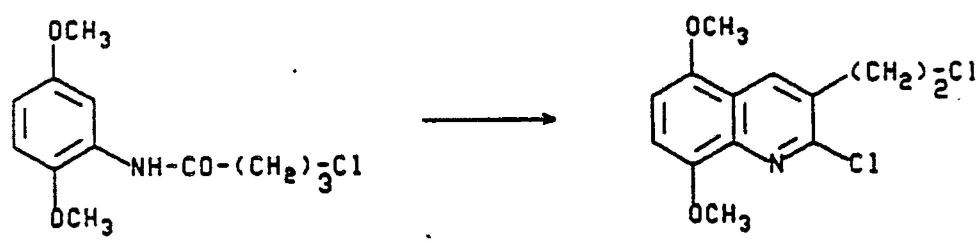

A solution of 2,5-dimethoxy-N-(4-chlorobutyroyl)-aniline (164g, 0.64 mole) in 300 mL of phosphorous oxychloride is cooled to 5° and 60 mL of dimethylformamide is added in portions over a 30-minute period. The solution is heated at 100° for 2 1/2 hours, cooled, and concentrated in vacuo. The residue is poured onto 2 L of ice water with stirring, and basified to pH 9 with ammonium hydroxide with ice cooling. Extraction of the reaction mixture with methylene chloride, drying, and concentration in vacuo affords the desired product, mp 108-109.5°.

## EXAMPLE 23

Preparation of 6-chloro-5-(2-chloroethyl)pyridine-2,3-dicarboxylic acid, dimethyl ester

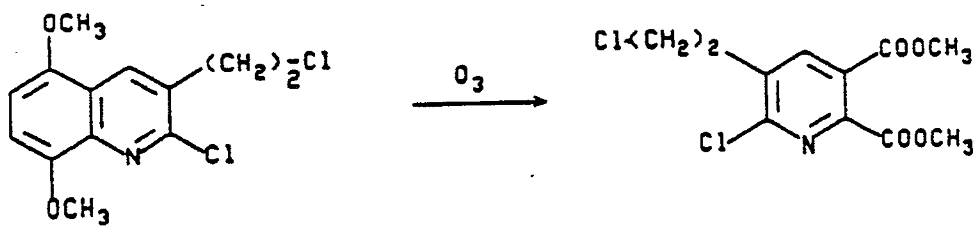


A solution of 2-chloro-3-(2-chloroethyl)-5,8-dimethoxyquinoline (22.0g, 0.077 mole) in 1 L methanol containing 80 mL of trimethylorthoformate and 2 mL concentrated sulfuric acid is stirred at room temperature while ozone is bubbled in until TLC indicates the disappearance of starting material. The reaction is concentrated *in vacuo*, and the residue is partitioned between ether and saturated aqueous bicarbonate. The organic layer is washed further with bicarbonate, then with aqueous bisulfite. Drying and concentration *in vacuo* affords the desired product as an oil.

## EXAMPLE 24

Preparation of 5-(2-chloroethyl)-pyridinedicarboxylic acid, dimethyl ester

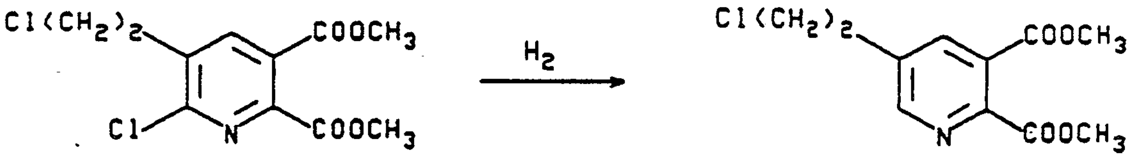


A mixture of 18g of 6-chloro-5-(2-chloroethyl)-pyridine-2,3-dicarboxylic acid, dimethyl ester, 1g of 10% palladium on carbon, and 7g of sodium acetate in 100 mL methanol is shaken in a hydrogenation apparatus under 15 psi for 23 hours. The reaction is then filtered through celite, and the catalyst is washed with a little methanol. The combined filtrates are concentrated *in vacuo*, and the residue is partitioned between methylene chloride and water. The organic phase is washed with saturated bicarbonate, dried, and concentrated *in vacuo*. The residue is chromatographed on silica gel using hexane-ethyl acetate mixtures to afford 8.9g of the desired product as an oil.

## EXAMPLE 25

Preparation of 5-vinyl-2,3-pyridinedicarboxylic acid, dimethyl ester

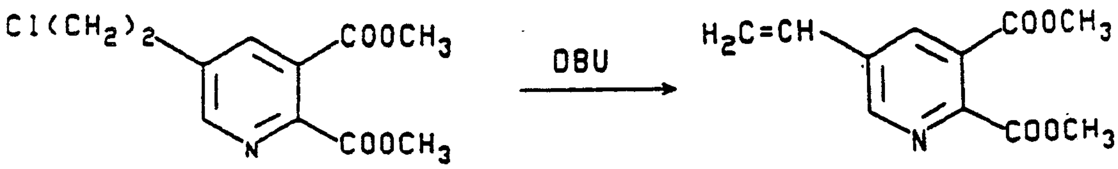


A solution of 2g of 5-(2-chloroethyl)-pyridine-2,3-dicarboxylic acid, dimethyl ester and 0.13 mL dia-

zabicycloundecane (DBU) in 40 mL of toluene is heated at reflux for 1 1/2 hours, cooled, and filtered. The filtrate is concentrated in vacuo, and the residue is chromatographed on silica gel using hexane-ethyl acetate mixtures to afford the desired product as a colorless oil.

## EXAMPLE 26

Preparation of 6-methyl-5-(N-methylacetamido)-pyridine-2,3-dicarboxylic acid, diethyl ester

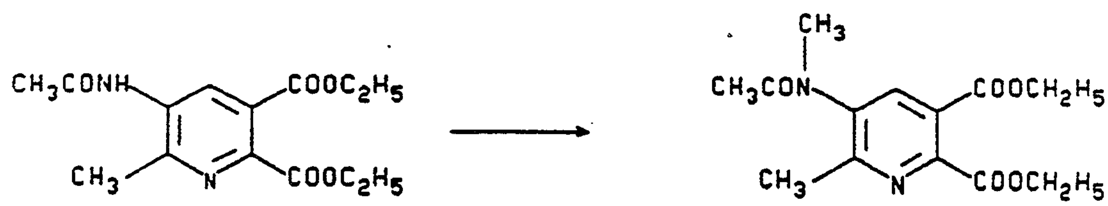

A stirred mixture of 2.94g of 5-acetamido-6-methylpyridine-2,3-dicarboxylic acid, diethyl ester, and 0.44g of 60% sodium hydride in 200 mL dry tetrahydrofuran is kept at room temperature for 1 hour, then 0.93 mL of methyl iodide is added, and the reaction is stirred for an additional 4 hours. The reaction is concentrated in vacuo, and the residue is slurried in water, the pH is adjusted to 3 with hydrochloric acid, and the mixture is extracted with methylenechloride. The organic phase is dried, concentrated in vacuo to afford the desired product as an oil.

Using essentially the same procedure, and substituting the appropriate acetamido- or hydroxyalkyl-pyridine precursor, one obtains the following:

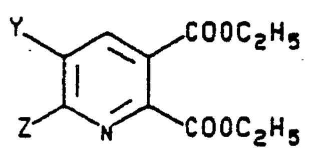

| Y | Z | mp |
|---|---|---|
| $CH_3$ | $CH_3OCH_2$- | oil |
| H | $CH_3OCH_2$- | oil |
| H | $CH_3CH(OCH_3)$- | oil |

## EXAMPLE 27

Preparation of 6-methyl-5-methylamino-pyridine-2,3-dicarboxylic acid, diethyl ester

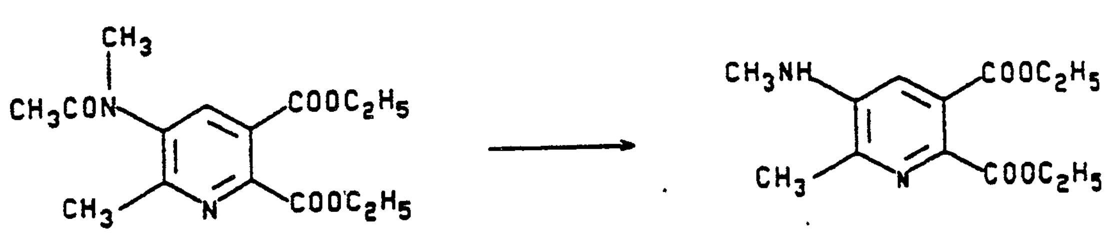

A solution of 90.6 g of 6-methyl-5-(N-methylacetamido)-pyridine-2,3-dicarboxylic acid, diethyl ester in 700 mL absolute ethanol is treated with 60 mL of concentrated hydrochloric acid, and the solution is heated at reflux for 17 hours. The reaction is cooled, 75 mL of concentrated hydrochloric acid is added, and the

reaction is heated at reflux for another 8 hours. The solution is concentrated in vacuo; the residue is stirred in water and basified to pH 8 using a saturated bicarbonate solution. The mixture is then extracted with methylene chloride, and the extracts are dried and concentrated in vacuo to afford the desired product as an oil.

EXAMPLE 28

Preparation of 6-methyl-2,3,5-pyridinetricarboxylic acid, 2,3-diethyl ester

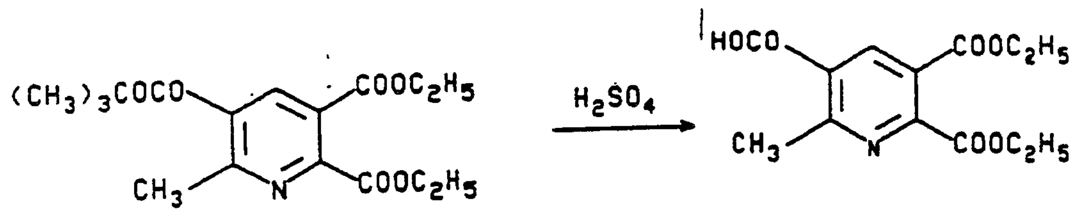


To 350 mL of concentrated sulfuric acid, cooled to 15° with mechanical stirring, is added 320 g of the starting material dropwise over a 70 minute period. The addition funnel is rinsed with ether, and the ether is added to the reaction mixture, which is allowed to stir at room temperature for three days. The reaction is poured onto an ether-ice mixture with efficient stirring, basified to pH 9 with concentrated ammonium hydroxide, and separated. The aqueous phase is acidified to pH 2 with concentrated hydrochloric acid and extracted with ethyl acetate. Drying and concentration in vacuo of the ethylacetate phase affords 231 g of the desired product as a low melting solid.

EXAMPLE 29

Preparation of 5-(hydroxymethyl)-6-methylpyridine-2,3-dicarboxylic acid, diethyl ester

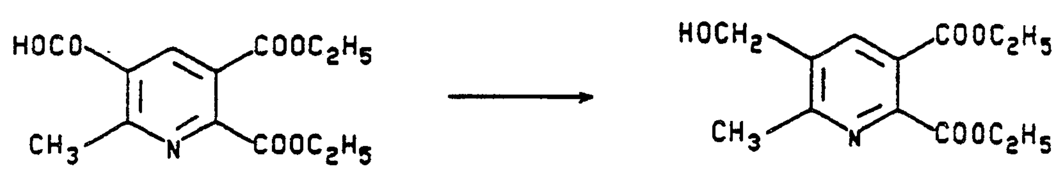

A solution of 464 g 6-methyl-2,3,5-pyridinetricarboxylic acid, 2,3-diethyl ester in 2.5 L toluene is cooled, under nitrogen, 5° with mechanical stirring while 200 mL of borane-dimethylsulfide complex is added dropwise over a one hour and 30 minute period. After stirring at room temperature for three days, the reaction is cooled to 10° and quenched with 250 mL of concentrated hydrochloric acid. A further 500 mL of 3M hydrochloric acid and 250 mL of ethyl acetate is added, and the reaction is vigorously stirred for three hours and 30 minutes. The organic layer can be filter chromatographed through silica gel and concentrated in vacuo to recover 96 g unreacted starting material. The aqueous layer is neutralized with 50% sodium hydroxide, extracted with ethyl acetate, concentrated in vacuo, and chromatographed on silica gel using hexane-ethyl acetate mixtures to afford 44 g of the desired product as an oil.

EXAMPLE 30

Preparation of 5-(methoxymethyl)-6-methylpyridine-2,3-dicarboxylic acid, diethyl ester

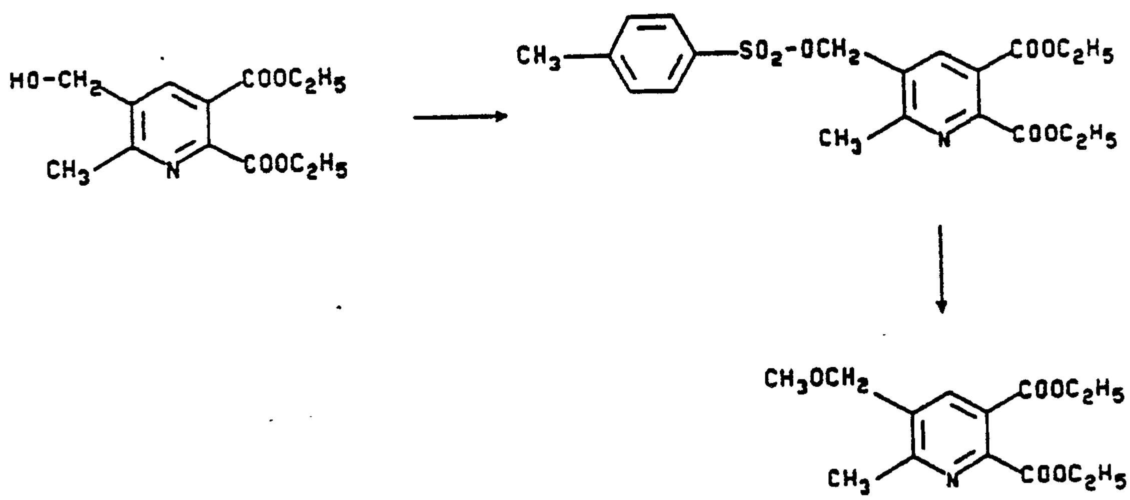

A solution of 5.0 g of 5-(hydroxymethyl)-6-methylpyridine-2,3-dicarboxylic acid, diethyl ester in 100 mL of methylene chloride is cooled to 0° and 20 mL of triethylamine and 4.0 g of p-toluenesulfonyl chloride is added. The reaction mixture is stirred at room temperature for 16 hours, filtered and concentrated in vacuo to afford the crude tosylate. This material is dissolved in 75 mL of ethanol; 3.8 g of 60% sodium hydride is added in small portions, and the reaction is stirred at room temperature for 24 hours. Acidification and concentration in vacuo affords the crude product as a mixture of methyl and ethyl esters.

Example 31

Preparation of 5-cyclohexyl-2,3-pyridine dicarboxylic acid.

A solution of 21.2g (0.07m) 5-cyclohexyl-2, 3-pyridine dicarboxylic acid diether ester in 100 ml dioxane is treated with 56 ml 5N NaOH solution and is stirred at room temperature for 72 hours. The solution is cooled in an ice bath, and the pH is adjusted to ~1 with 10% HCl to give 8.4g (48%) tan solid, mp 250-252° C, dec.

The following compound is prepared in similar fashion:

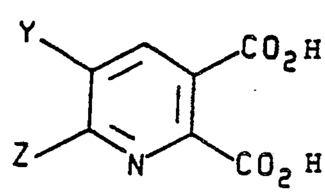

| Y | Z | mp |
|---|---|---|
| $CH_3CONH_2$ | $CH_3$ | 198-200°C (dec) |

Example 32

Preparation of 5-cyclohexyl-2-(4-isopropyl-4-methyl-5-oxo-2-imidazolin-2yl)-nicotinic acid.

A solution of 3.1g (0.013 mole) of 5-cyclohexyl-2.3-pyridine dicarboxylic anhydride and 1.75g (0.013

mole) of α-methyl γalinamide in 25ml THF is heated at reflux for 1 hour. The solution is stripped in vacuo and resultant oil is dissolved in 13ml 5N NaOH and is heated at 80° C for 2 hours. The reaction mixture is cooled in an ice-bath and the pH is adjusted to ~1 with 10% HCl. The resultant solid is filtered to afford 0.52g (11.5%) white solid, mp 207-209° C.

Using the same procedure the following compounds may be prepared:

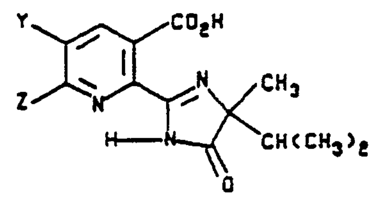

| Y | Z | mp |
|---|---|---|
| $CH_3SO_2N(CH_3)-$ | $CH_3$ | 213-216°C |
| $CH_3OCH_2-$ | $CH_3$ | 89-92°C |
| $CH_3$ | $CH_3OCH_2-$ | - |
| H | $CH_3OCH_2-$ | - |
| H | $CH_3CH(OCH_3)-$ | - |

## Example 33

Preparation of 2-(4-Isopropyl-4-methyl-5-oxo-2-imidazolin-2-yl)-5-(2-thienyl)-nicotinic acid, Methyl Ester.

A mixture of 2-(4-Isopropyl-4-methyl-5-oxo-2-imidazolin-2-yl)-5-(2-thienyl)-nicotinic acid (2.0g 0.00582 mole), acetic anhydride (0.71g, 0.00697 mole) and pyridine (0.46g, 0.00582 mole) in dry 1,2-dimethoxyethane (6.5 ml) is stirred at 25° C for three hours. The mixture is then concentrated to afford the corresponding mixture of tricyclics, which is dissolved in absolute methanol (150ml), to which sodium methoxide (0.5g, 0.0093 mole) is added. The solution is heated at reflux for 8 hours and allowed to stand overnight at 25° C. The solution is neutralized to pH 4 with acetic acid and concentrated to leave 3.12g of a gum, which is purified by chromatography on silica with chloroform as elutant and recrystallization from acetone-hexane solution to afford 0.49g of the desired methyl ester, mp 120-123.2° C, which is identified by NMR, and elemental analysis.

Using essentially the same procedure, and substituting the appropriate pyridine precursor and the appropriate nucleophile, the following were prepared:

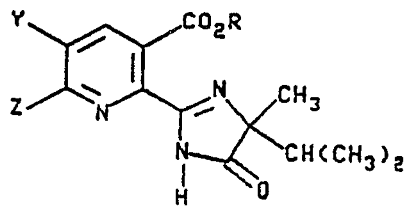

| Y | Z | R | mp |
|---|---|---|---|
| $NH_2$- | $CH_3$ | $CH_3$ | 169 -173°C |
| $CH_3CONH$- | $CH_3$ | $CH_3$ | 162 -165°C |
| $CH_3CONH$- | $CH_3$ | $CH_2$ | 195 -196°C |
| $CH_3CON(CH_3)$- | $CH_3$ | $CH_3$ | 191 -195°C |
| 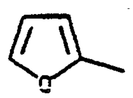 | H | $CH_3$ | 150.5-155.5°C |

Example 34

Preparation of 3-isopropyl-3-methyl-7-cyclohexyl-5H-Imidazo [1′,2′:1,2] pyrrolo [3.4-b] pyridine-3 (2H), 5-dione.

A solution of 1.35g Dicyclohexyl carbodiimide (0.006 mole) in 10ml $CH_2Cl_2$ is added to a solution of 2.0g 2-(4-isopropyl-4-methyl-5-oxo-2-imidazolin-2yl)-5-methoxy-methyl-nicotinic acid (0.006 mole) in 10ml $CH_2Cl_2$ and the mixture is stirred under $N_2$ at room temperature for 18 hours. The resultant solid is filtered and the filtrate is evaporated in vacuo to leave 1.9g (100%) of a clear yellow oil.

Example 35

Preparation of 5-acetyl-2,3-pyridine dicarboxylic acid, Diethyl Ester.

Combination of 4-dimethylamino-3-buten-2-one (33.8g, 0.313 mole) and diethyl ethoxymethylene-oxalacetate (70.bg. real, 0.29 mole) at 25°C gives a neat solution which rapidly warms to 48°C and then cools to 25°C within 3.5 hours. The red solution is held at 25°C overnight and then diluted with absolute ethanol (170 ml). Concentrated ammonium hydroxide (170ml of 30% aqueous) is added with cooling (10-32°C) over 20 minutes. The resulting solution is allowed to stand at 25°C for 4 hours, and is then concentrated in vacuo. The resulting residue is taken up in ether and the solution is washed repeatedly with water until the wash is only faintly colored. The ether solution is dried ($Na_2SO_4$) and concentrated to leave a red-brown oil, wt. 42.2g, which is chromatographed on silica with chloroform as elutant to afford 31.7g of the yellow diester, which is ~93 area % pure by gas-liquid chromatography.

Using the same procedure the following compounds are obtained:

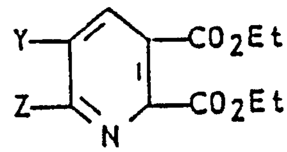

| Y | Z | mp |
|---|---|---|
| $CH_3CO$ | H | oil |
| $(CH_3)_3C-OCO$ | $CH_3$ | oil |
| $CH_3CO$ | $CH_3$ | oil |
| 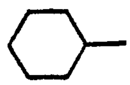 | H | oil |
| $CH_3$ | $CH_3$ | oil |
| H | $CH_3$ | oil |

Example 36

Preparation of Methyl-2(4-isopropyl-4-methyl-5-oxo-2-imidazolin-2-gl)-5-methoxy methyl-nicotinate.

A solution of Methyl-2(1-benzoyl-4-isopropyl-4-methyl-5-oxo-2-imidazolin-2-yl)-5-bromomethyl nicotinate (6.3g, 0.016 mole) in 50 ml absolute methanol is treated with 5.5 m. of a 25% sodium methoxide in methanol solution and is stirred overnight. The pH is adjusted to ~4 with conc. HCl and the solvent is removed in vacuo. The residue is chromatographed through $SiO_2$ with 10% THF/$CH_2Cl_2$ to ultimately give 0.63g 12.4%) of light yellow solid, mp. 88-89 °C.

Using essentially the same conditions as described above, and using the appropriate 5-(1-bromalkyl)-2-(4-isopropyl-4-methyl-5-oxo-2-imidazolin-2-yl)nicotinate and appropriate nucleophile, the following are prepared:

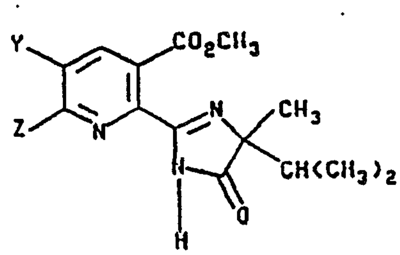

| Y | Z | mp |
|---|---|---|
| 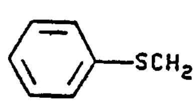 | H | 135 - 138° |

Example 37

Preparation of Cyclohexyl acetaldehyde, morpholine enamine.

A solution of 27.0g (0.214 mole) of cyclohexyl acetaldehyde and 18.6g morpholine (0.214 mole) in 150 ml toluene is treated with 2.0g (0.01m) toluene sulfonic acid and is heated to reflux for 4 hours under a water separator. After cooling the solution is filtered. Evaporation in vacuo gives 29.2g (65%) of a yellow liquid which is 94.6% pure by gas-liquid chromatography.

## Example 38

### Preparation of 2-(4-isopropyl-4-methyl-5-oxo-2-imidazolin-2-yl)-5-vinyl-nicotinic acid

A solution of 75g of the starting 2-[(1-carbamoyl-1,2-dimethylpropyl)carbamoyl]-5-(2-chloroethyl)nicotinic acid material in 20ml of SN sodium hydroxide is heated at 80°C for 2 hours. The solution is then cooled and acidified to pH 3 with hydrochloric acid. The product is filtered, dried, and recrystallized from ether-hexane to afford an off-white solid mp 183-184$^{C}$.

Using the same procedure the following compounds are prepared:

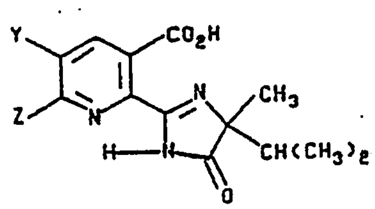

| | | |
|---|---|---|
| $CH_3-\overset{OH}{\underset{CH_3}{C}}-C\equiv C-$ | H | 158.0 - 160.0° |
| $CH_2=CH-$ | H | 183.0 - 184.0° |
| 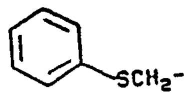 | H | 160.0 - 160.5° |
| $CH_3C\equiv C-$ | H | 185.0 - 189.0° |
| 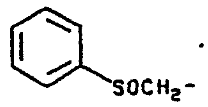 | H | 122.0 - 123.0° |
| 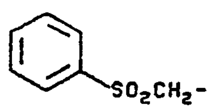 | H | 220.0 - 224.0° |
| $(CH_3O)_2CH-$ | H | oil |
| $CH_3OCH_2-$ | $CH_3O-$ | 180.0 - 181.0° |
| $CH_3\underset{OCH_3}{CH}-$ | H | glass |
| $CH_3\underset{OCH_3}{CH}-$ | $CH_3$ | 170.0 - 171.0° |
| $NH_2-$ | H | 210.0 - 220.0° |
| $CH_3CONH-$ | H | 275.0 - 278.0° (dec) |
| CHO | H | - |
| $CH_3OCONH-$ | $CH_3$ | 218.0 - 221.0° (dec) |
| $NH_2$ | $CH_3$ | 265.0 - 268.0° |
| $CH_3CONH-$ | $CH_3$ | 208.0 - 210.0° |
| $CH_3CO\underset{CH_3}{N}-$ | $CH_3$ | 196.0 - 200.0° |

Example 39

Preparation of 5-bromomethyl-2-(1-beazoyl-4-isopropyl-4-methyl-5-oxo-2-imidazolin-2-yl)-nicotinic acid, methyl ester

To a solution of 46.2g of starting Methyl 5-methyl-2-(1-benzoyl-4-isopropyl-4-methyl-5-oxo-2-imidazolin-2-yl)nicotinate material in 900ml carbon tetrachloride is added 23g of N-bromosuccinimide and 3.1g benzoyl peroxide, and the resulting solution is stirred overnight at 70° C. The reaction is cooled, diluted with $CU_2U_2$, and washed with 5% aqueous $Na_2S_2O_5$, then with water. Drying and concentration of the organic phase *in vacuo* affords a gum, which is chromatographed on silica using hexane-ethyl acetate mixtures to afford 19.6g of the title product, mp 88-119° C.

Using essentially the same conditions and substituting the appropriate nicotinate precursor, the following compound is obtained:

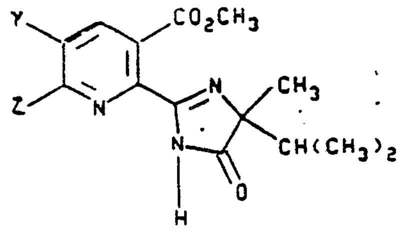

| Y | Z | mp |
|---|---|---|
| $Br^2CH-$ | H | 59-68°C |

Example 40

Preparation of Propargyl-2-(4-isopropyl-4-methyl-5-oxo-2-imidazolinyl-2yl)-5-methoxymethyl-nicotinate.

A solution of 1.9g (0.006 mole) of the starting 2-isopropyl-2-methyl-7-(methoxymethyl)-5H-imidazo-[1′,2′:1,2]pyrrolo[3,4-b]pyridine-3(2H), 5-dione material in 20ml THF and 0.9ml proparyl alcohol is treated with ~100mg 60% NaH and the resultant mixture is refluxed for 2 hours. The cooled mixture is adjusted to pH~4 with 10% HCl and the solvent is removed *in vacuo*. The residue is chromatographed through 100g $SiO_2$ with 10% $CH_2Cl_2/CH_3CN$ to leave 0.75g (38%) of a white solid mp. soften @80° C, 169-170° C.

Example 41

Preparation of 5-bromomethyl-2-(4-isopropyl-4-methyl-5-oxo-2-imidazolin-2-yl)nicotinic acid, methyl ester.

A solution of 33g starting Methyl 5-bromomethyl-2-(4-isopropyl-4-methyl-5-oxo-2-imidazolin-2-yl)-nicotinate material in 200ml methanol is stirred at room temperature, and CHCl is added in small portions over 24 hours, until TLC showed the disappearance of the starting material. The reaction mixture is then neutralized with saturated aqueous bicarbonate and concentrated *in vacuo* to afford the title product as a solid.

Example 42

Preemergence herbicidal evaluation of test compounds

The preemergence herbicidal activity of the compounds of the present invention is exemplified by the following tests in which the seeds of a variety of monocotyledonous and dicotyledonous plants are separately mixed with potting soil and planted on top of approximately one inch of soil in separate pint

cups. After planting, the cups are sprayed with the selected aqueous acetone solution containing test compound in sufficient quantity to provide the equivalent of about 0.016 to 8.0 kg per hectare of test compound per cup. The treated cups are then placed on greenhouse benches, watered and cared for in accordance with conventional greenhouse procedures. From four to give weeks after treatment, the tests are terminated and each cup is examined and rated according to the rating system set forth below. Data obtained are reported in table I below. Where more than one test is involved for a given compound, the data are averaged.

Plant species employed in these evaluations are reported by header abbreviation, common name and scientific name.

Compounds employed in this preemergence herbicidal evaluation and in the post-emergence evaluation in the following example are given a compound number and identified by name. Data in the table are reported by compound number.

***Herbicide Rating Scale**

Results of herbicide evaluation are expressed on a rating scale (0-9). The scale is based upon a visual observation of plant stand, vigor, malformation, size, chlorosis and overall plant appearance as compared with a control.

| RATING | MEANING | % Control (COMPARED TO CHECK) |
|---|---|---|
| 9 | Complete Kill | 100 |
| 8 | Approaching Complete Kill | 91-99 |
| 7 | Good Herbicidal Effect | 80-90 |
| 6 | Herbicidal Effect | 65-79 |
| 5 | Definite Injury | 45-64 |
| 4 | Injury | 30-44 |
| 3 | Moderate Effect | 16-29 |
| 2 | Slight Effect | 6-15 |
| 1 | Trace Effect | 1-5 |
| 0 | No Effect | 0 |
| P | "PGR" Effect | --- |
| X | Unable to Read Sample | --- |

| PLANT SPECIES EMPLOYED IN HERBICIDAL EVALUATIONS | | |
|---|---|---|
| Header Abb | Common Name | Scientific Name |
| BARNYARDGR | BARNYARDGRASS | ECHINOCHLOA CRUS-GALLI, (L)BEAU |
| FOXTAIL SP | FOXTAIL SPP. | SETARIA SPP. |
| P NUTSEDGE | NUTSEDGE, PURPLE | CYPERUS ROTUNDUS, L. |
| WILD OATS | OAT, WILD | AVENA FATUA, L. |
| QUACKGRASS | QUACKGRASS | AGROPYRON REPENS, (L)BEAUV. |
| FLD BINDWD | BINDWEED, FIELD (RHIZOME) | CONVOLVULUS ARVENSIS, L. |
| MATRUCARIA | MATRICARIA | MATRICARIA CHAMOMILLA, L |
| MRNGLRY SP | MORNINGGLORY SPP | IPONIEA SPP. |
| RAGWEED | RAGWEED, COMMON | AMBROSIA ARTEMISIIFOLIA, L. |
| VELVETLEAF | VELVETLEAF | ABUTILON THEOPHRASTI, MEDIC. |
| WHT FENMAN | WHEAT FENMAN | TRITICUM AESTIVUM, FENMAN |
| SUGARBEETS | SUGARBEETS | BETA VULGARIS, L. |
| CORN FIELD | CORN, FIELD | ZEA MAYS, L. |
| COTTON | COTTON | GOSSYPIUM HIRSUTUM, L. |
| RICE, NATO | RICE, NATO | ORYZA SATIVA, L. CV. NATO |
| SOYBEAN BR | SOYBEAN, BRAGG | GLYCINE MAX(L) MERR. CV. BRAGG |

## COMPOUNDS EVALUATED AS HERBICIDAL AGENTS

| Compound No | |
|---|---|
| 1 | 2-(4-isopropyl-4-methyl-5-oxo-2-imidazolin-2-yl)-5-[(methylthio)methyl]nicotinic acid |
| 2 | 2-(4-isopropyl-4-methyl-5-oxo-2-imidazolin-2-yl)-5-(methoxymethyl)nicotinic acid |
| 3 | 5-cyclohexyl-2-(4-isopropyl-4-methyl-5-oxo-2-imidazolin-2-yl)nicotinic acid |
| 4 | 2-(4-isopropyl-4-methyl-5-oxo-2-imidazolin-2-yl)-5-amino-6-methylnicotinic acid |
| 5 | 5-(cyanomethyl)-2-(4-isopropyl-(4-methyl-5-oxo-2-imidazolin-2-yl)nicotinic acid |
| 6 | 5-cyclopropyl-2-(4-isopropyl-4-methyl-5-oxo-2-imidazolin-2-yl)nicotinic acid |
| 7 | 5-formyl-2-(4-isopropyl-4-methyl-5-oxo-2-imidazolin-2-yl)nicotinic acid, 5-(dimethyl acetal) |
| 8 | 5-[(allyloxy)methyl]-2-(4-isopropyl-4-methyl-5-oxo-2-imidazolin-2-yl)nicotinic acid |

| Compound No | |
|---|---|
| 9 | 5-(sec-butoxymethyl)-2-(4-isopropyl-4-methyl-5-oxo-2-imidazolin-2-yl)nicotinic acid |
| 10 | 5-[(2-propynyloxy)methyl]-2-(4-isopropyl-4-methyl-5-oxo-2-imidazolin-2-yl)-nicotinic acid |
| 11 | 2-(4-isopropyl-4-methyl-5-oxo-2-imidazolin-2-yl)-5-(ethoxymethyl)nicotinic acid |
| 12 | 5-(methoxymethyl)-2-(1-benzoyl-4-isopropyl-4-methyl-5-oxo-2-imidazolin-2-yl)nicotinic acid |
| 13 | 5-formyl-2-(4-isopropyl-4-methyl-5-oxo-2-imidazolin-2-yl)nicotinic acid |
| 14 | 2-(4-isopropyl-4-methyl-5-oxo-2-imidazolin-2-yl)-5-(methoxymethyl)-6-methylnicotinic acid |
| 15 | 2-(4-isopropyl-4-methyl-5-oxo-2-imidazolin-2-yl)-6-(methoxymethyl)nicotinic acid |

# TABLE I

PRE-EMERGENCE TESTS -- RATES IN KG/HA

| Compound | RATE | BARNY ARDGR | FOXTA IL SP | P NUT SEDGE | WILD OATS | QUACK GRASS | FLD B INDWD | MATRI CARIA | MRNGL RY SP | RAGWE ED | VELVE TLEAF | WHT F ENMAN | SUGAR BEETS | CORN FIELD | COTTO N | RICE, NATO | SOYBE AN BR |
|---|---|---|---|---|---|---|---|---|---|---|---|---|---|---|---|---|---|
| 1 | .500 | 0.0 | 2.0 | 8.0 | 0.0 | 4.0 | 9.0 | 2.0 | 7.0 | 0.0 | 6.0 | 0.0 | 9.0 | 0.0 | 4.0 | 2.0 | 0.0 |
| | .250 | 0.0 | 0.0 | 4.0 | 0.0 | 3.0 | 6.0 | 0.0 | 4.0 | 0.0 | 4.0 | 0.0 | 8.0 | 0.0 | 2.0 | 1.0 | 0.0 |
| | .125 | 0.0 | 0.0 | 2.0 | 0.0 | 1.0 | 4.0 | 0.0 | 2.0 | 0.0 | 4.0 | 0.0 | 8.0 | 0.0 | 0.0 | 1.0 | 0.0 |
| | .063 | 0.0 | 0.0 | 0.0 | 0.0 | 0.0 | 2.0 | 0.0 | 0.0 | 0.0 | 0.0 | 0.0 | 4.0 | 0.0 | 0.0 | 1.0 | 0.0 |
| | .032 | 0.0 | 0.0 | 0.0 | 0.0 | 0.0 | 0.0 | 0.0 | 0.0 | 0.0 | 0.0 | 0.0 | 2.0 | 0.0 | 0.0 | 0.0 | 0.0 |
| | .016 | 0.0 | 0.0 | 0.0 | 0.0 | 0.0 | 0.0 | 0.0 | 0.0 | 0.0 | 0.0 | 0.0 | 2.0 | 0.0 | 0.0 | 0.0 | 0.0 |
| 2 | .500 | 9.0 | 9.0 | 9.0 | 9.0 | 9.0 | 9.0 | 9.0 | 9.0 | 7.0 | 9.0 | 9.0 | 9.0 | 9.0 | 9.0 | 9.0 | 3.0 |
| | .250 | 7.0 | 9.0 | 9.0 | 9.0 | 9.0 | 9.0 | 7.0 | 8.5 | 6.5 | 8.0 | 9.0 | 9.0 | 8.8 | 8.3 | 9.0 | 2.0 |
| | .125 | 6.0 | 9.0 | 8.7 | 8.5 | 8.7 | 9.0 | 4.0 | 8.0 | 7.0 | 8.0 | 9.0 | 9.0 | 8.5 | 8.3 | 9.0 | 1.0 |
| | .063 | 5.0* | 9.0 | 8.0 | 8.0 | 9.0 | 9.0 | 4.0 | 7.7 | 4.7 | 8.0 | 9.0 | 9.0 | 8.3 | 7.8 | 9.0 | 0.0 |
| | .032 | 1.7 | 3.0 | 7.7 | 8.0 | 8.7 | 6.0 | 0.0 | 4.3 | 2.3* | 7.0 | 8.0 | 9.0 | 7.7 | 6.3 | 7.0 | 1.0 |
| | .016 | 0.0 | 0.0 | 6.3 | 6.5 | 8.7 | 6.0 | 0.0 | 1.7 | 1.3 | 6.8 | 6.0 | 8.5 | 5.8 | 5.5* | 6.0 | 0.0 |
| 3 | .500 | 7.0 | 8.0 | 0.0 | 6.0 | 9.0 | 9.0 | 0.0 | 2.0 | 0.0 | 8.0 | 6.0 | 8.0 | 7.0 | 2.0 | 8.0 | 2.0 |
| | .250 | 7.0 | 2.0 | 0.0 | 6.0 | 6.0 | 7.0 | 0.0 | 1.0 | 0.0 | 7.0 | 6.0 | 8.0 | 2.0 | 2.0 | 7.0 | 1.0 |
| | .125 | 2.0 | 1.0 | 0.0 | 1.0 | 2.0 | 4.0 | 0.0 | 0.0 | 0.0 | 3.0 | 1.0 | 7.0 | 2.0 | 2.0 | 7.0 | 0.0 |
| | .063 | 1.0 | 0.0 | 0.0 | 0.0 | 1.0 | 2.0 | 0.0 | 0.0 | 0.0 | 1.0 | 0.0 | 6.0 | 1.0 | 1.0 | 4.0 | 0.0 |
| | .032 | 0.0 | 0.0 | 0.0 | 0.0 | 0.0 | 0.0 | 0.0 | 0.0 | 0.0 | 0.0 | 0.0 | 4.0 | 1.0 | 0.0 | 2.0 | 0.0 |
| | .016 | 0.0 | 0.0 | 0.0 | 0.0 | 0.0 | 0.0 | 0.0 | 0.0 | 0.0 | 0.0 | 0.0 | 2.0 | 0.0 | 0.0 | 0.0 | 0.0 |
| 4 | .500 | 6.7* | 9.0 | 9.0 | 9.0 | 9.0 | 9.0 | 0.0 | 9.0 | 4.7 | 9.0 | 9.0 | 9.0 | 9.0 | 9.0 | 9.0 | 9.0 |
| | .250 | 4.0* | 8.3 | 8.7 | 8.3 | 9.0 | 8.7 | 0.0 | 8.3 | 2.3 | 8.7 | 9.0 | 9.0 | 9.0 | 8.7 | 9.0 | 7.7 |
| | .125 | 3.0 | 7.0 | 8.3 | 8.7 | 9.0 | 8.3 | 0.0 | 7.7 | 1.3 | 8.0 | 8.7 | 9.0 | 8.3 | 8.0 | 7.0 | 7.0 |
| | .063 | 1.7 | 5.7 | 7.7 | 8.0 | 9.0 | 7.7 | 0.0 | 6.7 | 0.0 | 6.7 | 7.7 | 8.3 | 6.7* | 8.3 | 6.0 | 5.3 |
| | .032 | 1.0 | 0.7 | 5.7* | 4.7 | 3.0* | 5.7* | 0.0 | 3.7* | 0.0 | 4.7 | 5.3 | 6.7* | 5.0 | 7.3 | 4.0 | 3.0 |
| | .016 | 1.0 | 0.0 | 2.0 | 2.0 | 1.0 | 2.0 | 0.0 | 1.3 | 0.0 | 2.7 | 2.0 | 5.0* | 2.3 | 5.7 | 4.0 | 1.7 |

TABLE I (Continued)

PRE-EMERGENCE TESTS -- RATES IN KG/HA

| Compound | RATE | BARNY ARDGR | FOXTA IL SP | P NUT SEDGE | WILD OATS | QUACK GRASS | FLD B INDWD | MATRI CARIA | MRNGL RY SP | RAGWE ED | VELVE TLEAF | WHT F ENMAN | SUGAR BEETS | CORN FIELD | COTTO N | RICE, NATO | SOYBE AN BR |
|---|---|---|---|---|---|---|---|---|---|---|---|---|---|---|---|---|---|
| 5 | .500 | 9.0 | 9.0 | 9.0 | 9.0 | 9.0 | 9.0 | 4.0 | 8.0 | 9.0 | 8.0 | 9.0 | 9.0 | 9.0 | 9.0 | 9.0 | 4.0 |
| | .250 | 4.0 | 9.0 | 9.0 | 9.0 | 9.0 | 9.0 | 4.0 | 8.0 | 4.0 | 8.0 | 9.0 | 9.0 | 8.0 | 9.0 | 9.0 | 4.0 |
| | .125 | 2.0 | 8.0 | 9.0 | 4.0 | 0.0 | 9.0 | 2.0 | 7.0 | 0.0 | 8.0 | 8.0 | 9.0 | 4.0 | 9.0 | 9.0 | 4.0 |
| | .063 | 0.0 | 2.0 | 8.0 | 2.0 | 0.0 | 9.0 | 2.0 | 0.0 | 0.0 | 7.0 | 3.0 | 9.0 | 2.0 | 4.0 | 8.0 | 1.0 |
| | .032 | 0.0 | 2.0 | 2.0 | 0.0 | 0.0 | 0.0 | 0.0 | 0.0 | 0.0 | 2.0 | 1.0 | 8.0 | 1.0 | 2.0 | 4.0 | 0.0 |
| | .016 | 0.0 | 0.0 | 2.0 | 0.0 | 0.0 | 0.0 | 0.0 | 0.0 | 0.0 | 0.0 | 1.0 | 7.0 | 0.0 | 2.0 | 4.0 | 0.0 |
| 6 | .500 | 9.0 | 9.0 | 9.0 | 9.0 | 9.0 | 9.0 | 9.0 | 9.0 | 9.0 | 9.0 | 9.0 | 9.0 | 7.0 | 9.0 | 9.0 | 4.0 |
| | .250 | 9.0 | 9.0 | 9.0 | 9.0 | 9.0 | 9.0 | 9.0 | 9.0 | 9.0 | 9.0 | 9.0 | 9.0 | 7.0 | 9.0 | 9.0 | |
| | .125 | 9.0 | 9.0 | 9.0 | 9.0 | 9.0 | 9.0 | 9.0 | 8.0 | 9.0 | 9.0 | 8.0 | 9.0 | 7.0 | 9.0 | 9.0 | 4.0 |
| | .063 | 6.0 | 9.0 | 8.0 | 7.0 | 9.0 | 9.0 | 9.0 | 8.0 | 9.0 | 8.0 | 6.0 | 9.0 | 6.0 | 9.0 | 9.0 | |
| | .032 | 4.0 | 9.0 | 8.0 | 4.0 | 4.0 | 9.0 | 2.0 | 8.0 | 7.0 | 8.0 | 4.0 | 9.0 | 5.0 | 9.0 | 9.0 | 4.0 |
| | .016 | 0.0 | 7.0 | 7.0 | 2.0 | | 9.0 | 2.0 | 7.0 | 7.0 | 7.0 | 2.0 | 9.0 | 5.0 | 9.0 | 9.0 | 3.0 |
| 7 | .500 | 9.0 | 9.0 | 9.0 | 9.0 | 9.0 | 9.0 | | 8.0 | 2.0 | 9.0 | 9.0 | 9.0 | 9.0 | 9.0 | | 3.0 |
| | .250 | 8.5 | 8.0 | 6.0 | 9.0 | 9.0 | 9.0 | | 8.0 | 6.0 | 8.0 | 9.0 | 9.0 | 9.0 | 8.5 | | 1.0 |
| | .125 | 8.5 | 9.0 | 2.0 | 9.0 | 9.0 | 9.0 | | 8.0 | 5.0* | 8.0 | 9.0 | 9.0 | 9.0 | 7.5 | | 1.0 |
| | .063 | 5.0 | 8.0 | 3.5 | 9.0 | 8.5 | 9.0 | | 7.0 | 1.5 | 6.7 | 8.0 | 8.0 | 8.0 | 6.0 | | 0.0 |
| | .032 | 4.0 | 6.0 | 0.0 | 8.0 | 9.0 | 6.0 | | 3.5 | 2.0 | 6.3 | 8.0 | 8.0 | 7.5 | 4.5 | | 0.0 |
| | .016 | 0.0 | | 0.0 | 5.0 | 8.0 | | | 0.0 | 2.0 | 5.5 | | 7.0 | 6.0 | 1.0 | | |
| 8 | .500 | 8.0 | 9.0 | 9.0 | 9.0 | | 9.0 | | 8.0 | 8.0 | 9.0 | 8.0 | 9.0 | 9.0 | 9.0 | | 6.0 |
| | .250 | 9.0 | 9.0 | 9.0 | 9.0 | | 9.0 | | 8.0 | 8.0 | 8.0 | 8.0 | 9.0 | 9.0 | 9.0 | | 4.0 |
| | .125 | 6.0 | 9.0 | 8.0 | 9.0 | | 9.0 | | 8.0 | 7.0 | 8.0 | 8.0 | 9.0 | 8.0 | 9.0 | | 3.0 |
| | .063 | 4.0 | 6.0 | 7.0 | 9.0 | | 8.0 | | 7.0 | 6.0 | 8.0 | 8.0 | 9.0 | 7.0 | 9.0 | | 2.0 |
| | .032 | 0.0 | 4.0 | 2.0 | 7.0 | | 0.0 | | 4.0 | 2.0 | 6.0 | 6.0 | 8.0 | 6.0 | 7.0 | | 2.0 |
| | .016 | 0.0 | 2.0 | 0.0 | 7.0 | | 0.0 | | 2.0 | 0.0 | 6.0 | 6.0 | 7.0 | 4.0 | 7.0 | | |

TABLE I (Continued)

PRE-EMERGENCE TESTS -- RATES IN KG/HA

| Compound | RATE | BARNY ARDGR | FOXTA IL SP | P NUT SEDGE | WILD OATS | QUACK GRASS | FLD B INDWD | MATRI CARIA | MRNGL RY SP | RAGWE ED | VELVE TLEAF | WHT F ENMAN | SUGAR BEETS | CORN FIELD | COTTO N | RICE, NATO | SOYBE AN BR |
|---|---|---|---|---|---|---|---|---|---|---|---|---|---|---|---|---|---|
| 9 | .500 | 9.0 | 9.0 | 9.0 | 9.0 | | 9.0 | | 9.0 | 8.0 | 9.0 | 8.0 | 9.0 | 8.0 | 9.0 | | 7.0 |
| | .250 | 8.0 | 9.0 | 7.0 | 9.0 | | 9.0 | | 9.0 | 9.0 | 9.0 | 7.0 | 9.0 | 8.0 | 9.0 | | 6.0 |
| | .125 | 4.0 | 8.0 | 9.0 | 9.0 | | 9.0 | | 9.0 | 4.0 | 8.0 | 7.0 | 9.0 | 8.0 | 8.0 | | 4.0 |
| | .063 | 4.0 | 8.0 | 7.0 | 9.0 | | 7.0 | | 7.0 | 2.0 | 7.0 | 6.0 | 9.0 | 4.0 | 7.0 | | 4.0 |
| | .032 | 2.0 | 7.0 | 8.0 | 7.0 | 2.0 | 2.0 | | 6.0 | 0.0 | 7.0 | 7.0 | 9.0 | 4.0 | 7.0 | | 6.0 |
| | .016 | 2.0 | 2.0 | 6.0 | 7.0 | 2.0 | 2.0 | | 6.0 | 0.0 | 6.0 | 7.0 | 9.0 | 3.0 | 7.0 | | 7.0 |
| 10 | .500 | 9.0 | 9.0 | 9.0 | 9.0 | | 9.0 | | 9.0 | 9.0 | 8.0 | 9.0 | 9.0 | 9.0 | 9.0 | | 7.0 |
| | .250 | 8.0 | 9.0 | 9.0 | 9.0 | | 9.0 | | 8.0 | 8.0 | 9.0 | 9.0 | 9.0 | 9.0 | 9.0 | | 4.0 |
| | .125 | 8.0 | 9.0 | 9.0 | 9.0 | | 9.0 | | 8.0 | 8.0 | 9.0 | 9.0 | 9.0 | 9.0 | 9.0 | | 4.0 |
| | .063 | 9.0 | 9.0 | 7.0 | 9.0 | | 8.0 | | 7.0 | 7.0 | 7.0 | 9.0 | 9.0 | 8.0 | 9.0 | | 3.0 |
| | .032 | 2.0 | 9.0 | 8.0 | 8.0 | | 9.0 | | 2.0 | 2.0 | 7.0 | 8.0 | 8.0 | 7.0 | 9.0 | | 3.0 |
| | .016 | 2.0 | 8.0 | 4.0 | 7.0 | | 7.0 | | 4.0 | 2.0 | 7.0 | 7.0 | 8.0 | 6.0 | 9.0 | | 2.0 |
| 11 | .500 | 9.0 | 9.0 | 9.0 | 9.0 | | 9.0 | | 9.0 | 8.0 | 9.0 | 9.0 | 9.0 | 9.0 | 9.0 | | 6.0 |
| | .250 | 9.0 | 9.0 | 7.0 | 9.0 | | 9.0 | | 8.0 | 8.0 | 9.0 | 9.0 | 9.0 | 9.0 | 9.0 | | 3.0 |
| | .125 | 6.0 | 9.0 | 7.0 | 9.0 | | 9.0 | | 8.0 | 7.0 | 9.0 | 9.0 | 9.0 | 8.0 | 8.0 | | 2.0 |
| | .063 | 7.0 | 6.0 | 6.0 | 9.0 | | 9.0 | | 6.0 | 7.0 | 8.0 | 9.0 | 9.0 | 7.0 | 8.0 | | 2.0 |
| | .032 | 2.0 | 2.0 | 2.0 | 8.0 | | 0.0 | | 2.0 | 2.0 | 7.0 | 8.0 | 9.0 | 4.0 | 7.0 | | 1.0 |
| | .016 | 0.0 | 2.0 | 2.0 | 7.0 | | 0.0 | | 2.0 | 0.0 | 6.0 | 4.0 | 8.0 | 4.0 | 6.0 | | |
| 12 | .500 | 8.0 | 9.0 | 9.0 | 9.0 | 9.0 | 9.0 | | 8.0 | 6.0 | 8.0 | 9.0 | 9.0 | 9.0 | 9.0 | | 2.0 |
| | .250 | 6.0 | 8.0 | 9.0 | 7.0 | 9.0 | 9.0 | | 8.0 | 6.0 | 8.0 | 8.0 | 9.0 | 9.0 | 9.0 | | 2.0 |
| | .125 | 8.0 | 9.0 | 8.0 | 9.0 | 8.0 | 9.0 | | 4.0 | 3.0 | 8.0 | 9.0 | 9.0 | 8.0 | 8.0 | | 1.0 |
| | .063 | 2.0 | 4.0 | 4.0 | 4.0 | 9.0 | 8.0 | | 2.0 | 1.0 | 7.0 | 8.0 | 9.0 | 7.0 | 6.0 | | 1.0 |
| | .032 | 2.0 | 4.0 | 4.0 | 7.0 | 4.0 | 3.0 | | 0.0 | 1.0 | 4.0 | 4.0 | 8.0 | 3.0 | 8.0 | | 3.0 |
| | .016 | 0.0 | 4.0 | 2.0 | 4.0 | 0.0 | 1.0 | | 0.0 | 0.0 | 2.0 | 2.0 | 4.0 | 3.0 | 2.0 | | 2.0 |

TABLE I (Continued)

PRE-EMERGENCE TESTS -- RATES IN KG/HA

| Compound | RATE | BARNY ARDGR | FOXTA IL SP | P NUT SEDGE | WILD OATS | QUACK GRASS | FLD B INDWD | MATRI CARIA | MRNGL RY SP | RAGWE ED | VELVE TLEAF | WHT F ENMAN | SUGAR BEETS | CORN FIELD | COTTO N | RICE, NATO | SOYBE AN BR |
|---|---|---|---|---|---|---|---|---|---|---|---|---|---|---|---|---|---|
| 13 | .500 | 0.0 | 0.0 | 0.0 | 0.0 | 0.0 | 0.0 | | 0.0 | 0.0 | 0.0 | 0.0 | 0.0 | 0.0 | 0.0 | | 0.0 |
| | .250 | 0.0 | 0.0 | 0.0 | 0.0 | 0.0 | 0.0 | | 0.0 | 0.0 | 0.0 | 0.0 | 0.0 | 0.0 | 0.0 | | 0.0 |
| | .125 | 0.0 | 0.0 | 0.0 | 0.0 | 0.0 | 0.0 | | 0.0 | 0.0 | 0.0 | 0.0 | 0.0 | 0.0 | 0.0 | | 0.0 |
| | .063 | 0.0 | 0.0 | 0.0 | 0.0 | 0.0 | 0.0 | | 0.0 | 0.0 | 0.0 | 0.0 | 0.0 | 0.0 | 0.0 | | 0.0 |
| | .032 | 0.0 | 0.0 | 0.0 | 0.0 | 0.0 | 0.0 | | 0.0 | 0.0 | 0.0 | 0.0 | 0.0 | 0.0 | 0.0 | | 0.0 |
| 14 | .500 | 9.0 | 7.0 | 9.0 | 9.0 | 9.0 | 9.0 | | 9.0 | 4.0 | 8.0 | 9.0 | 9.0 | 9.0 | 9.0 | | 5.0 |
| | .250 | 4.0 | 7.0 | 8.0 | 9.0 | 9.0 | 9.0 | | 7.0 | 4.0 | 8.0 | 9.0 | 9.0 | 9.0 | 9.0 | | 2.0 |
| | .125 | 2.0 | 7.0 | 4.0 | 9.0 | 9.0 | 9.0 | | 7.0 | 2.0 | 6.0 | 9.0 | 7.0 | 8.0 | 8.0 | | 2.0 |
| | .063 | 2.0 | 4.0 | 4.0 | 8.0 | 9.0 | 9.0 | | 4.0 | 0.0 | 5.0 | 7.0 | 7.0 | 8.0 | 6.0 | | 1.0 |
| | .032 | 2.0 | 4.0 | 4.0 | 7.0 | 6.0 | 8.0 | | 2.0 | 0.0 | 5.0 | 7.0 | 6.0 | 7.0 | 6.0 | | 1.0 |
| 15 | .500 | 2.0 | 9.0 | 9.0 | 9.0 | 9.0 | 9.0 | | 9.0 | 6.0 | 9.0 | 9.0 | 9.0 | 9.0 | 9.0 | | 9.0 |
| | .250 | 2.0 | 9.0 | 9.0 | 9.0 | 9.0 | 9.0 | | 9.0 | 2.0 | 8.0 | 9.0 | 9.0 | 9.0 | 9.0 | | 9.0 |
| | .125 | 0.0 | 9.0 | 9.0 | 7.0 | 9.0 | 9.0 | | 8.0 | 2.0 | 8.0 | 9.0 | 9.0 | 9.0 | 9.0 | | 8.0 |
| | .063 | 0.0 | 6.0 | 9.0 | 7.0 | 9.0 | 9.0 | | 7.0 | 0.0 | 7.0 | 9.0 | 9.0 | 9.0 | 8.0 | | 8.0 |
| | .032 | 0.0 | 0.0 | 7.0 | 2.0 | | | | 7.0 | 0.0 | 7.0 | 8.0 | | 9.0 | 8.0 | | 7.0 |
| | .016 | 0.0 | 0.0 | 9.0 | 2.0 | | | | | 0.0 | 7.0 | 7.0 | | 7.0 | 7.0 | | 6.0 |

## EXAMPLE 43

Postemergence herbicidal evaluation of test compounds

The postemergence herbicidal activity of the compounds of the present invention is determined by the following tests, wherein a variety of monocotyledonous and dicotyledonous plants are treated with test compounds dispersed in aqueous acetone mixtures. In the tests, seedling plants are grown in jiffy flats for about two weeks. The test compounds are dispersed in 50/50 acetone/water mixtures containing 0.5% TWEEN® 20, a polyoxyethylene sorbitan monolaurate surfactant of Atlas Chemical Industries, in sufficient quantities to provide the equivalent of about 0.16 kg to 8.0 kg per hectare of active compound when applied to the plants through a spray nozzle operating at 40 psig for a predetermined time. After spraying, the plants are placed on greenhouse benches and are cared for in the usual manner, commensurate with conventional greenhouse practices. From four to give weeks after treatment, the seedling plants, are examined and rated according to the rating system provided in Example 36 above. The data obtained are recorded in Table II below. The compounds evaluated are reported by compound number given in Example 38.

TABLE II

POST-EMERGENCE TESTS -- RATES IN KG/HA

| Compound | RATE | BARNY ARDGR | FOXTA IL SP | P NUT SEDGE | WILD OATS | QUACK GRASS | FLD B INDWD | MATRI CARIA | MRNGL RY SP | RAGWE ED | VELVE TLEAF | WHT F ENMAN | SUGAR BEETS | CORN FIELD | COTTO N | RICE, NATO | SOYBE AN BR |
|---|---|---|---|---|---|---|---|---|---|---|---|---|---|---|---|---|---|
| 1 | .500 | 9.0 | 9.0 | 7.0 | 9.0 | 6.0 | 8.0 | 3.0 | 9.0 | 7.0 | 9.0 | 6.0 | 9.0 | 6.0 | 9.0 | 8.0 | 3.0 |
| | .250 | 9.0 | 7.0 | 7.0 | 8.0 | 7.0 | 7.0 | 1.0 | 7.0 | 6.0 | 7.0 | 3.0 | 8.0 | 3.0 | 8.0 | 7.0 | 2.0 |
| | .125 | 5.0 | 6.0 | 2.0 | 8.0 | 3.0 | 7.0 | 0.0 | 4.0 | 2.0 | 6.0 | 1.0 | 5.0 | 1.0 | 7.0 | 3.0 | 1.0 |
| | .063 | 2.0 | 0.0 | 1.0 | 4.0 | 1.0 | 6.0 | 0.0 | 0.0 | 1.0 | 2.0 | 0.0 | 2.0 | 0.0 | 2.0 | 2.0 | 0.0 |
| | .032 | 0.0 | 0.0 | 0.0 | 2.0 | 0.0 | 2.0 | 0.0 | 0.0 | 0.0 | 1.0 | 0.0 | 2.0 | 0.0 | 1.0 | 2.0 | 0.0 |
| 2 | .500 | 9.0 | 9.0 | 9.0 | 9.0 | 9.0 | 9.0 | 9.0 | 9.0 | 9.0 | 9.0 | 9.0 | 9.0 | 9.0 | 9.0 | 9.0 | 5.0 |
| | .250 | 8.5 | 9.0 | 9.0 | 9.0 | 9.0 | 9.0 | 9.0 | 8.6 | 8.3 | 9.0 | 9.0 | 9.0 | 9.0 | 9.0 | 9.0 | 4.8 |
| | .125 | 8.3 | 9.0 | 8.3 | 9.0 | 9.0 | 9.0 | 9.0 | 8.3 | 7.6 | 8.7 | 9.0 | 8.5 | 9.0 | 9.0 | 9.0 | 3.4 |
| | .063 | 8.3 | 9.0 | 7.3 | 8.5 | 9.0 | 9.0 | 8.0 | 8.4 | 7.0 | 8.4 | 9.0 | 8.5 | 8.8 | 8.5 | 9.0 | 2.4 |
| | .032 | 8.3 | 9.0 | 6.0 | 8.5 | 8.3 | 9.0 | 6.0 | 7.8 | 6.3 | 7.6 | 9.0 | 8.5 | 8.8 | 8.5 | 9.0 | 0.8 |
| | .016 | 8.0 | | 1.5 | 7.0 | 8.5 | | | 6.9 | 4.0* | 6.3 | | 8.0 | 8.3 | 7.0 | | 0.5 |
| 3 | .500 | 9.0 | 9.0 | 0.0 | 4.0 | 2.0 | 9.0 | | 2.0 | 2.0 | 7.0 | 3.0 | 9.0 | 7.0 | 4.0 | 7.0 | 1.0 |
| | .250 | 2.0 | 8.0 | 0.0 | 0.0 | 1.0 | 9.0 | | 2.0 | 2.0 | 4.0 | 1.0 | 9.0 | 7.0 | 3.0 | 6.0 | 0.0 |
| | .125 | 0.0 | 2.0 | 0.0 | 0.0 | 0.0 | 4.0 | | 0.0 | 2.0 | 2.0 | 1.0 | 4.0 | 7.0 | 2.0 | 6.0 | 0.0 |
| | .063 | 0.0 | 0.0 | 0.0 | 0.0 | 0.0 | 2.0 | | 0.0 | 0.0 | 2.0 | 0.0 | 4.0 | 4.0 | 1.0 | 2.0 | 0.0 |
| | .032 | 0.0 | 0.0 | 0.0 | 0.0 | 0.0 | 0.0 | | 0.0 | 0.0 | 0.0 | 0.0 | 2.0 | 4.0 | 0.0 | 2.0 | 0.0 |
| 4 | .500 | 8.3 | 9.0 | 7.3 | 8.7 | 9.0 | 9.0 | 8.0 | 8.7 | 7.3 | 9.0 | 9.0 | 9.0 | 9.0 | 8.7 | 9.0 | 8.3 |
| | .250 | 7.3 | 9.0 | 6.7 | 8.7 | 8.3 | 8.7 | 0.0 | 8.3 | 3.7 | 8.0 | 8.7 | 9.0 | 9.0 | 8.7 | 9.0 | 8.0 |
| | .125 | 6.3* | 9.0 | 6.3 | 9.0 | 8.7 | 9.0 | 0.0 | 8.0 | 3.3 | 8.0 | 8.7 | 9.0 | 8.0 | 8.3 | 9.0 | 7.3 |
| | .063 | 5.7* | 8.3 | 6.0 | 5.7* | 7.0 | 6.7 | 0.0 | 5.0 | 1.3 | 6.0 | 8.7 | 9.0 | 6.7* | 7.3 | 8.0 | 6.3 |
| | .032 | 6.5 | 7.0 | 3.3 | 5.3* | 4.0* | 7.0 | 0.0 | 5.0 | 0.7 | 4.3 | 8.7 | 9.0 | 6.0* | 7.3 | 7.0 | 5.7 |

TABLE II (Continued)

POST-EMERGENCE TESTS -- RATES IN KG/HA

| Compound | RATE | BARNY ARDGR | FOXTA IL SP | P NUT SEDGE | WILD OATS | QUACK GRASS | FLD B INDWD | MATRI CARIA | MRNGL RY SP | RAGWE ED | VELVE TLEAF | WHT F ENMAN | SUGAR BEETS | CORN FIELD | COTTO N | RICE, NATO | SOYBE AN BR |
|---|---|---|---|---|---|---|---|---|---|---|---|---|---|---|---|---|---|
| 7 | .500 | 9.0 | 9.0 | 7.5 | 9.0 | 9.0 | 9.0 | | 9.0 | 8.5 | 9.0 | 9.0 | 9.0 | 9.0 | 9.0 | 9.0 | 5.0 |
| | .250 | 9.0 | 9.0 | 6.7 | 8.7 | 9.0 | 9.0 | | 8.5 | 7.5 | 8.4 | 9.0 | 9.0 | 9.0 | 9.0 | 9.0 | 3.7 |
| | .125 | 9.0 | 9.0 | 4.3 | 8.7 | 9.0 | 9.0 | | 8.5 | 7.3 | 7.8 | 9.0 | 9.0 | 9.0 | 8.5 | 9.0 | 2.3 |
| | .063 | 9.0 | 9.0 | 3.3 | 8.7 | 8.7 | 9.0 | | 8.3 | 5.5* | 7.6 | 9.0 | 9.0 | 9.0 | 8.0 | 9.0 | 1.3 |
| | .032 | 8.3 | 8.5 | 2.0 | 8.3 | 8.3 | 8.5 | | 7.8 | 3.0* | 5.6 | 8.5 | 9.0 | 9.0 | 7.5 | 9.0 | 0.7 |
| | .016 | 7.0 | | 0.0 | 4.0 | 6.0 | | | 7.5 | 0.5 | 2.0 | | | 8.0 | | | 0.0 |
| 8 | .500 | 9.0 | 9.0 | 7.0 | 9.0 | 9.0 | 9.0 | | 8.5 | 7.0 | 8.0 | 9.0 | 9.0 | 9.0 | 8.0 | | 4.0 |
| | .250 | 8.0 | 9.0 | 6.0 | 9.0 | 9.0 | 9.0 | | 8.0 | 6.5 | 8.0 | 9.0 | 9.0 | 9.0 | 8.0 | | 3.5 |
| | .125 | 9.0 | 9.0 | 6.0 | 9.0 | 9.0 | 9.0 | | 8.0 | 3.5 | 6.0 | 9.0 | 9.0 | 9.0 | 8.0 | | 2.5 |
| | .063 | 4.0 | 9.0 | 3.0 | 6.0 | 4.0 | 9.0 | | 7.0 | 1.5 | 4.0 | 9.0 | 9.0 | 8.5 | 7.0 | | 2.0 |
| | .032 | 3.0 | 8.0 | 0.0 | 6.0 | 6.0 | 7.0 | | 6.0 | 1.0 | 2.5 | 7.0 | 9.0 | 8.0 | 7.0 | | 2.0 |
| | .016 | | | | | | | | 0.0 | 0.0 | 0.0 | | | 4.0 | | | 0.0 |
| 9 | .500 | 8.0 | 9.0 | 8.0 | 9.0 | 9.0 | 9.0 | | 9.0 | 4.0 | 8.0 | 9.0 | 9.0 | 9.0 | 8.0 | | 4.0 |
| | .250 | 4.0 | 8.0 | 2.0 | 2.0 | 9.0 | 4.0 | | 8.0 | 2.0 | 6.0 | 8.0 | 9.0 | 6.0 | 7.0 | | 2.0 |
| | .125 | 6.0 | 8.0 | 2.0 | 2.0 | 9.0 | 7.0 | | 6.0 | 0.0 | 2.0 | 8.0 | 9.0 | 5.0 | 7.0 | | 1.0 |
| | .063 | 2.0 | 7.0 | 0.0 | 2.0 | 4.0 | | | 0.0 | 0.0 | 0.0 | 2.0 | 8.0 | 2.0 | 3.0 | | 1.0 |
| | .032 | 0.0 | 4.0 | 0.0 | 0.0 | 2.0 | 6.0 | | 0.0 | 0.0 | 0.0 | 2.0 | 8.0 | 1.0 | 2.0 | | 1.0 |
| 10 | .500 | 9.0 | 9.0 | 8.0 | 9.0 | 9.0 | 9.0 | | 9.0 | 8.0 | 9.0 | 9.0 | 9.0 | 9.0 | 9.0 | | 7.0 |
| | .250 | 9.0 | 9.0 | 8.0 | 9.0 | 9.0 | 9.0 | | 8.0 | 8.0 | 9.0 | 9.0 | 9.0 | 9.0 | 8.0 | | 4.0 |
| | .125 | 9.0 | 9.0 | 7.0 | 9.0 | 9.0 | 9.0 | | 7.0 | 7.0 | 7.0 | 9.0 | 9.0 | 9.0 | 8.0 | | 4.0 |
| | .063 | 4.0 | 9.0 | 4.0 | 6.0 | 9.0 | 9.0 | | 7.0 | 2.0 | 4.0 | 9.0 | 9.0 | 9.0 | 7.0 | | 2.0 |
| | .032 | 4.0 | 7.0 | 2.0 | 6.0 | 8.0 | 6.0 | | 6.0 | 0.0 | 2.0 | 8.0 | 9.0 | 9.0 | 7.0 | | 1.0 |

## TABLE II (Continued)

POST-EMERGENCE TESTS -- RATES IN LB/ACRE

| Compound | RATE | BARNY ARDGR | FOXTA IL SP | P NUT SEDGE | WILD OATS | QUACK GRASS | FLD B INDWD | MATRI CARIA | MRNGL RY SP | RAGWE ED | VELVE TLEAF | WHT F ENMAN | SUGAR BEETS | CORN FIELD | COTTO N | RICE, NATO | SOYBE AN BR |
|---|---|---|---|---|---|---|---|---|---|---|---|---|---|---|---|---|---|
| 11 | .500 | 9.0 | 9.0 | 9.0 | 9.0 | 9.0 | 9.0 | | 9.0 | 9.0 | 9.0 | 9.0 | 9.0 | 9.0 | 9.0 | | 5.0 |
| | .250 | 9.0 | 9.0 | 8.0 | 9.0 | 9.0 | 9.0 | | 8.5 | 8.0 | 9.0 | 9.0 | 9.0 | 9.0 | 8.0 | | 5.0 |
| | .125 | 9.0 | 9.0 | 7.0 | 9.0 | 9.0 | 9.0 | | 8.5 | 8.0 | 8.5 | 9.0 | 9.0 | 9.0 | 8.0 | | 3.5 |
| | .063 | 8.0 | 9.0 | 7.0 | 9.0 | 9.0 | 9.0 | | 8.0 | 5.0 | 7.0 | 9.0 | 9.0 | 9.0 | 8.0 | | 2.5 |
| | .032 | 4.0 | 8.0 | 4.0 | 9.0 | 9.0 | 9.0 | | 8.0 | 4.0 | 6.0 | 9.0 | 9.0 | 9.0 | 7.0 | | 1.5 |
| | .016 | | | | | | | | 8.0 | 0.0 | 5.0 | | | 7.0 | | | 0.0 |
| 12 | .500 | 9.0 | 9.0 | 2.0 | 9.0 | 9.0 | 9.0 | | 7.0 | 4.0 | 8.0 | 9.0 | 9.0 | 9.0 | 8.0 | | 0.0 |
| | .250 | 6.0 | 9.0 | 0.0 | 9.0 | 8.0 | 9.0 | | 7.0 | 0.0 | 8.0 | 9.0 | 8.0 | 9.0 | 8.0 | | 0.0 |
| | .125 | 0.0 | 9.0 | 0.0 | 7.0 | 7.0 | 9.0 | | 7.0 | 0.0 | 6.0 | 7.0 | 8.0 | 9.0 | 7.0 | | 0.0 |
| | .063 | 0.0 | 7.0 | 0.0 | 4.0 | 0.0 | 9.0 | | 2.0 | 0.0 | 4.0 | 2.0 | 4.0 | 7.0 | 6.0 | | 0.0 |
| | .032 | 0.0 | 4.0 | 0.0 | 2.0 | 0.0 | 4.0 | | 2.0 | 0.0 | 4.0 | 2.0 | 2.0 | 2.0 | 4.0 | | 0.0 |
| 13 | .500 | 2.0 | 7.0 | 2.0 | 4.0 | 2.0 | 9.0 | | 9.0 | 2.0 | 6.0 | 6.0 | 9.0 | 7.0 | 6.0 | | 4.0 |
| | .250 | 0.0 | 7.0 | 0.0 | 2.0 | 0.0 | 7.0 | | 9.0 | 0.0 | 4.0 | 2.0 | 9.0 | 7.0 | 6.0 | | 3.0 |
| | .125 | 0.0 | 4.0 | 0.0 | 0.0 | 0.0 | 7.0 | | 8.0 | 0.0 | 2.0 | 1.0 | 8.0 | 3.0 | 4.0 | | 1.0 |
| | .063 | 0.0 | 4.0 | 0.0 | 0.0 | 0.0 | 4.0 | | 7.0 | 0.0 | 2.0 | 0.0 | 6.0 | 2.0 | 3.0 | | 1.0 |
| | .032 | 0.0 | 2.0 | 0.0 | 0.0 | 0.0 | 2.0 | | 0.0 | 0.0 | 0.0 | 0.0 | 4.0 | 2.0 | 2.0 | | 1.0 |
| 14 | .500 | 9.0 | 9.0 | 6.0 | 9.0 | 9.0 | 9.0 | | 9.0 | 8.0 | 9.0 | 9.0 | 9.0 | 9.0 | 9.0 | | 9.0 |
| | .250 | 9.0 | 9.0 | 6.0 | 9.0 | 9.0 | 9.0 | | 8.5 | 7.5 | 8.0 | 9.0 | 9.0 | 9.0 | 9.0 | | 5.0 |
| | .125 | 8.0 | 9.0 | 6.0 | 9.0 | 9.0 | 9.0 | | 8.0 | 5.5 | 7.5 | 9.0 | 9.0 | 9.0 | 9.0 | | 4.0 |
| | .063 | 9.0 | 9.0 | 4.0 | 9.0 | 8.0 | 9.0 | | 8.0 | 4.5 | 6.0 | 9.0 | 9.0 | 9.0 | 8.0 | | 2.5 |
| | .032 | 7.0 | 9.0 | 4.0 | 9.0 | 9.0 | 9.0 | | 7.5 | 3.5 | 4.5 | 9.0 | 9.0 | 9.0 | 8.0 | | 1.5 |
| | .016 | | | | | | | | 7.0 | 1.0 | 2.0 | | | 9.0 | | | 1.0 |

TABLE II (Continued)

POST-EMERGENCE TESTS -- RATES IN LB/ACRE

| Compound | RATE | BARNY ARDGR | FOXTA IL SP | P NUT SEDGE | WILD OATS | QUACK GRASS | FLD B INDWD | MATRI CARIA | MRNGL RY SP | RAGWE ED | VELVE TLEAF | WHT F ENMAN | SUGAR BEETS | CORN FIELD | COTTO N | RICE, NATO | SOYBE AN BR |
|---|---|---|---|---|---|---|---|---|---|---|---|---|---|---|---|---|---|
| 15 | .500 | 9.0 | 9.0 | 9.0 | 8.0 | 9.0 | 8.0 | | 9.0 | 7.0 | 9.0 | 9.0 | 9.0 | 9.0 | 9.0 | | 9.0 |
| | .250 | 6.0 | 9.0 | 9.0 | 9.0 | 9.0 | 9.0 | | 9.0 | 6.0 | 9.0 | 9.0 | 9.0 | 9.0 | 9.0 | | 9.0 |
| | .125 | 8.0 | 9.0 | 8.0 | 9.0 | 9.0 | 9.0 | | 9.0 | 4.0 | 9.0 | 9.0 | 9.0 | 9.0 | 8.0 | | 8.0 |
| | .063 | 9.0 | 9.0 | 8.0 | 9.0 | 8.0 | 8.0 | | 8.0 | 4.0 | 7.0 | 9.0 | 9.0 | 9.0 | 8.0 | | 8.0 |
| | .032 | 2.0 | 8.0 | 7.0 | 7.0 | 8.0 | 8.0 | | 8.0 | 2.0 | 7.0 | 9.0 | 9.0 | 9.0 | 7.0 | | 7.0 |

## Claims

1. A compound having the structure:

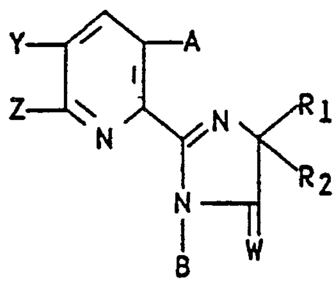

(I)

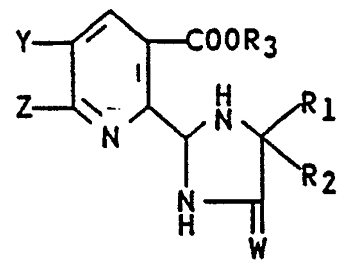

(II)

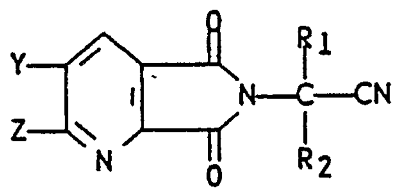

(III)

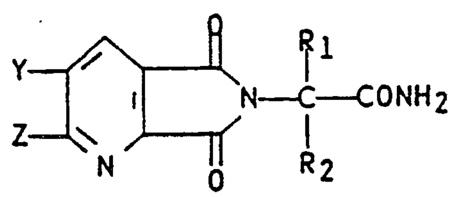

(IV)

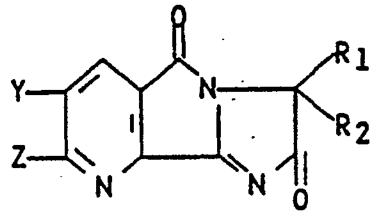

(V)

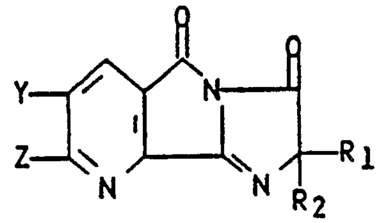

(VI)

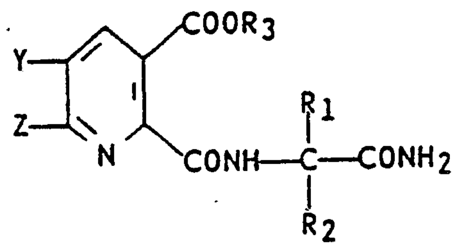

(VII)

wherein

$R_1$ is $C_1$-$C_4$ alkyl;

$R_2$ is $C_1$-$C_4$ or $C_3$-$C_6$ cycloalkyl; and when $R_1$ and $R_2$ are taken together with the carbon to which they are attached they may represent $C_3$-$C_6$ cycloalkyl optionally substituted with methyl;

A is $COOR_3$, $CONHR_6$, CHO, $CH_2OH$, $COCH_3$, $COC_6H_5$, CN, $CH_3$, CH=NOH, $CH_2COOH$, CONHOH, $CH_2CH_2COOH$, $CHR_8OH$,

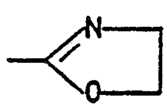

or

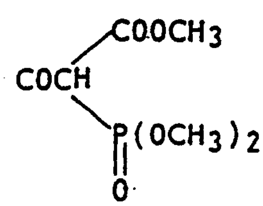

;

$R_3$ is hydrogen,

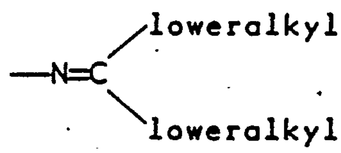

$C_1$-$C_{12}$ alkyl optionally substituted with one of the following groups: $C_1$-$C_3$ alkoxy, $C_1$-$C_3$ alkylthio, halogen, hydroxyl, $C_3$-$C_6$ cycloalkyl, benzyloxy, furyl, phenyl, halophenyl, loweralkylphenyl, loweralkoxyphenyl, nitrophenyl, carboxyl, loweralkoxycarbonyl, cyano or triloweralkylammonium halide;
$C_3$-$C_{12}$ alkenyl optionally substituted with one of the following groups: $C_1$-$C_3$ alkoxy, phenyl, halogen or loweralkoxycarbonyl or with two $C_1$-$C_3$ alkoxy groups or two halogen groups;
$C_3$-$C_6$ cycloalkyl optionally substituted with one or two $C_1$-$C_3$ alkyl groups;
$C_3$-$C_{16}$ alkynyl optionally substituted with one or two $C_1$-$C_3$ alkyl groups; or
a cation;
$R_6$ is hydrogen, hydroxyl, $C_3$-alkenyl, $C_3$-alkynyl or $C_1$-$C_4$ alkyl optionally substituted with one hydroxyl or one chlorine group;
B is H, $COR_4$ or $SO_2R_5$, provided that when B is $COR_4$ or $SO_2R_5$; A is $COOR_3$ in which $R_3$ is other than H, or a cation, $CH_3$ or CN; W is O; and Y and Z are not alkylamino, hydroxyl, or hydroxyloweralkyl;
$R_4$ is $C_1$-$C_{11}$ alkyl, chloromethyl or phenyl optionally substituted with one chloro, one nitro or one methoxy group;
$R_5$ is $C_1$-$C_4$ alkyl or phenyl optionally substituted with one methyl group;
W is O or S;
$R_3$ is $C_1$-$C_4$ alkyl or phenyl;
Y and Z are each hydrogen, methyl;
$C_2$-$C_5$ straight or branched alkynyl optionally substituted with hydroxy;
$C_3$-$C_6$ cycloalkyl optionally interrupted by 1 oxygen, sulfur, amino, or $C_1$-$C_4$ alkylamino; oxiranyl optionally substituted by one or two $C_1$-$C_4$ alkyl;
$C_1$-$C_4$ alkyl substituted with one or more of the following groups:
$C_1$-$C_4$ alkoxy optionally substituted with phenyl, thienyl, furyl, cyclopropyl, tetrahydrofuryl, $C_1$-$C_4$ alkoxy, $C_1$-$C_4$ dialkylamino, $C_1$-$C_4$ alkylamino, $C_1$-$C_4$ trialkylammonium, or 1 to 3 halogens; with the proviso that when the substituent on the $C_1$-$C_4$ alkoxy group is $C_1$-$C_4$ alkoxy, $C_1$-$C_4$ alkylamino, $C_1$-$C_4$ dialkylamino, or $C_1$-$C_4$ trialkylammonium, the respective heteroatoms are separated by at least 2 carbon atoms;
$C_1$-$C_4$ alkenyloxy optionally substituted with 1 to 3 halogens;
$C_1$-$C_4$ alkynyloxy optionally substituted with 1 to 3 halogens;
$C_3$-$C_6$ cycloalkoxy;
phenyl thio;
$C_1$-$C_4$ alkylthio optionally substituted with phenyl or 1 to 3 halogens;
$C_1$-$C_4$ alkylsulfinyl optionally substituted with phenyl or 1 to 3 halogens;
$C_1$-$C_4$ alkylsulfonyl optionally substituted with phenyl or 1 to 3 halogens;
$C_1$-$C_4$ alkylamino optionally substituted on carbon by phenyl or 1 to 3 halogens;
cyano;
phenylamino, optionally substituted in the ring by 1 to 3 halogens, $C_1$-$C_4$ lower alkyl, or $C_1$-$C_4$ lower alkoxy;
phenoxy, optionally substituted in the ring by 1 to 3 halogens, $C_1$-$C_4$ lower alkyl, or $C_1$-$C_4$ lower alkoxy;
formyl;
amino optionally substituted with $C_1$-$C_4$ alkylsulfonyl and $C_1$-$C_4$ alkyl, amino, $C_1$-$C_4$ monoalkylamino, or $C_1$-$C_4$ dialkly amino;
Provided that at least one of Y and Z is:
$C_2$-$C_5$ straight or branched alkynyl optionally substituted with hydroxy;
$C_3$-$C_6$ cycloalkyl optionally interrupted by one oxygen, sulfur, amino, or $C_1$-$C_4$ alkylamino; oxiranyl optionally substituted by one or two $C_1$-$C_4$ alkyl;
$C_1$-$C_4$ alkyl substituted with one or more of the following groups:
$C_1$-$C_4$ alkoxy optionally substituted with phenyl, thienyl, furyl, cyclopropyl, tetrahydrofuryl, $C_1$-$C_4$ alkoxy, $C_1$-$C_4$ dialkylamino, $C_1$-$C_4$ alkylamino, $C_1$-$C_4$ trialkylammonium, or 1 to 3 halogens; with the proviso that when the substituent on the $C_1$-$C_4$ alkoxy group is $C_1$-$C_4$ alkoxy, $C_1$-$C_4$ alkylamino, $C_1$-$C_4$ dialkylamino, or $C_1$-$C_4$ trialkylammonium, the respective heteroatoms are separated by at least 2 carbon atoms;
$C_1$-$C_4$ alkenyloxy optionally substituted with 1 to 3 halogens;
$C_1$-$C_4$ alkynyl oxy optionally substituted with 1 to 3 halogens;
$C_3$-$C_6$ cycloalkoxy; phenyl thio;

$C_1$-$C_4$ alkylthio optionally substituted with phenyl or 1 to 3 halogens;
$C_1$-$C_4$ alkylsulfinyl optionally substituted with phenyl or 1 to 3 halogens;
$C_1$-$C_4$ alkylsulfonyl optionally substituted with phenyl or 1 to 3 halogens;
$C_1$-$C_4$ alkylamino optionally substituted on carbon by phenyl or 1 to 3 halogens;
cyano;
phenylamino, optionally substituted in the ring by 1 to 3 halogens; $C_1$-$C_4$ lower alkyl, or $C_1$-$C_4$ lower alkoxy; formyl; optionally substituted in the ring by 1 to 3 halogens; $C_1$-$C_4$ lower alkyl, or $C_1$-$C_4$ lower alkoxy; formyl;
amino optionally substituted with $C_1$-$C_4$ alkylsulfonyl and $C_1$-$C_2$ alkyl, amino, $C_1$-$C_4$ monoalkylamino, or $C_1$-$C_4$ dialkyl amino;
Provided also that when Y is hydroxy, Z cannot be hydrogen;
the N-oxides thereof when W is O provided that $R_3$ cannot be unsaturated alkyl and Y and Z cannot be alkylthioalkyl, alkylaminoalkyl, dialkylaminoalkyl, trialkylammoniumalkyl, amino, 2(or 4)-pyridyloxy, alkoxyamino or unsaturated alkyl;
the optical isomers thereof when $R_1$ and $R_2$ are not the same;
the tautomers thereof; and
the acid addition salts thereof except when $R_3$ is a cation.

2. A compound according to claim 1, wherein the compound is 2-(4-isopropyl-4-methyl-5-oxo-2-imidazolin-2-yl)nicotinic acid and the esters and salts thereof.

3. A compound according to claim 1 wherein the compound is 2-(4-isopropyl-4-methyl-5-oxo-imidazolin-2-yl)-5-(methoxymethyl) nicotinic acid and the esters and salts thereof.

4. A compound according to claim 1, wherein the compound is 2-(4-isopropyl-4-methyl-5-oxo-2-imidazolin-2-yl)-5-(ethoxymethyl)nicotinic acid and esters and salts thereof.

5. A compound according to claim 1, wherein the compound is 2-(4-isopropyl-4-methyl-5-oxo-2-imidazolin-2-yl)-5-(methoxymethyl)-6-methylnicotinic acid and the esters and salts thereof.

6. A compound according to claim 1, wherein the compound is 5-formyl-2-(4-isopropyl-4-methyl-5-oxo-2-imidazolin-2-yl)nicotinic acid, 5-(dimethyl acetal) and the esters and salts thereof.

7. A compound according to claim 1, wherein the compound is 5-formyl-2-(4-isopropyl-4-methyl-5-oxo-2-imidazolin-2-yl)nicotinic acid and the esters and salts thereof.

8. A compound according to claim 1, wherein the compound is 2-(4-isopropyl-4-methyl-5-oxo-2-imidazolin-2-yl)-5-amino-6-methylnicotinic acid and the esters and salts thereof.

9. A compound according to claim 1, wherein the compound is 2-(4-isopropyl-4-methyl-5-oxo-2-imidazolin-2-yl)-5-[(methylthio)methyl]nicotinic acid and the esters and salts thereof.

10. A process for the preparation of a compound of the formula:

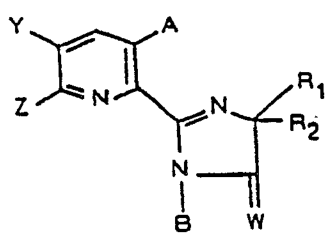

wherein $R_1$, $R_2$, W, Y, and Z are as defined in Claim 1, A is $COOR_3$, wherein $R_3$ is as defined in Claim 1, or $CONHR_6$ wherein $R_6$ is as defined in Claim 1 comprising reacting a compound having the formula:

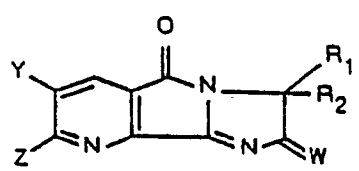

or

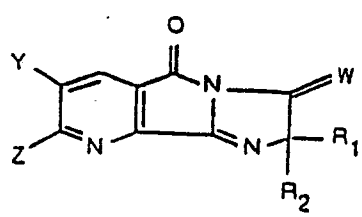

wherein $R_1$, $R_2$, W, Y, and Z are as defined in Claim 1 with

(a) at least one equivalent of an alcohol of formula $R_3OH$ and an alkali metal alkoxide $R_3O^-M^+$ where $R_3$ is as described above and M is an alkali metal, alone or in the presence of an aprotic solvent at a temperature between 20 °C and 50 °C, whereby the desired product, in which A is $COOR_3$ and $R_3$ is as defined above, is obtained or

(b) at least one equivalent of an amine of formula $R_6NH_2$, wherein $R_6$ is as defined in Claim 7, alone or in the presence of an aprotic solvent, at a temperature between 80 °C and 125 °C, whereby the desired product, in which A is $CONHR_6$ wherein $R_6$ is as defined above, is formed.